(19) Europäisches Patentamt
European Patent Office
Office européen des brevets

(11) Publication number: 0 067 685
B1

(12) # EUROPEAN PATENT SPECIFICATION

(45) Date of publication of patent specification: 05.11.86

(21) Application number: 82303036.6

(22) Date of filing: 11.06.82

(51) Int. Cl.[4]: C 07 D 277/74, C 07 D 513/04, A 61 K 31/425, A 61 K 31/44 // (C07D513/04, 277:00, 221:00)

(54) **Alpha-phenyl-2-(aza)benzothiazolylthioglycolic acids and derivatives thereof.**

(30) Priority: 15.06.81 US 273826
19.01.82 GB 8201475

(43) Date of publication of application:
22.12.82 Bulletin 82/51

(45) Publication of the grant of the patent:
05.11.86 Bulletin 86/45

(84) Designated Contracting States:
AT BE CH DE FR IT LI LU NL SE

(56) References cited:
EP-A-0 021 773
EP-A-0 030 632
EP-A-0 055 248

(73) Proprietor: AMERICAN HOME PRODUCTS CORPORATION
685, Third Avenue
New York, New York 10017 (US)

(72) Inventor: Wei, Peter Hsing-Lien
430, Ridge Lane Springfield
Delaware Pennsylvania 19064 (US)
Inventor: Gregory, Francis Joseph
11, Cypress Lane Berwyn
Chester Pennsylvania 19312 (US)

(74) Representative: Porter, Graham Ronald et al
C/O John Wyeth & Brother Limited
Huntercombe Lane South Taplow
Maidenhead Berkshire, SL6 0PH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description for the Contracting States: AT BE CH DE FR IT LI NL SE**

The invention relates to specific α-phenyl-2-(aza)benzothiazolylthioglycolic acids, derivatives thereof and pharmacologically acceptable salts thereof. The compounds are useful as modulators of the immune response. The specific use of other compounds is pointed out below.

In recent years, the rapid upsurge in immunological research has brought about a greater appreciation and understanding of the complexities of the immune response. While the traditional overall view of the immune system remains, new discoveries have radically changed some thinking about the details of the system. Thus, the immune system is still divided into humoral immunity, populated with B cells and responsible for antibody formation, and cell-mediated immunity, populated with T cells and responsible for the rejection of organ transplants or skin grafts, as well as the defence mechanism against various foreign biological matter and endogenous neoplastic growths.

It is only in the last decade or so, however, that the concept has been accepted that different cell populations interact in the induction and expression of both humoral and cell-mediated immunity. Thus, subpopulations of B cells and T cells have been described, such as for example "suppressor and "helper" T cells. In a number of animal models, it has been postulated that the helper T cells act in the induction of a complete antibody response to many antigens, whereas T suppressor cells are capable of preventing or terminating such responses. It is now believed that positive and negative cellular interactions control the ultimate degree of immune response. So, it is believed that any given immune response is regulated, and that the degree and mode of regulation may ultimately explain the various reactions, diseases, and disorders which are the manifestations of the operation of the organism's immune system.

The T cell subpopulations of suppressor and helper T cells have been implicated in a number of immune response manifestations. Thus, the loss of suppressor T cells is now believed to be a major factor in such autoimmune connective tissue disease as systemic lupus erythematosus. Moreover, in the latter case, as well as in probable impaired immune system responses such as rheumatoid arthritis, it is believed the helper T cells exacerbate the condition.

Also, the theory has been advanced that T suppressor cell hypofunctioning, resulting in inadequate T—B cell cooperation in the immune response, with continuous B cell stimulation and subsequent antibody production may be the cause of the production of antigen-antibody complexes which are the causative agents of renal and inflammatory processes in arthritis and autoimmune diseases.

Thus, it is now apparent that a number of lymphopoietic disorders are undoubtedly associated with abnormalities of T cell and especially suppressor cell function. The loss of suppressor function is at least an early event in certain immune response diseases and is a disease-perpetuating mechanism in other. The loss of suppressor function probably leads to excessive lymphoid cell proliferation and may significantly contribute to lymphoproliferative disorders. The conditions created thereby may be exacerbated by helper T cells.

The role of immunomodulatory agents in the treatment of immune diseases and disorders, as well as in the attempt to prolong the life of organ transplants and skin grafts, has been to suppress the immune response, especially of cell-mediated immunity. By suppressing the cell-mediated immune response, it is possible to delay and possibly prevent the host organism from rejecting a skin graft or organ transplant, or the graft from immunologically rejecting the host (graft vs. host reaction). Similarly, enhancing or reinstituting suppressor function by immunomodulator therapy is a beneficial course of treatment for autoimmune and probable autoimmune diseases and disorders. Immunotherapy is also being recognised as an important aspect of the treatment, especially chemotherapeutic, of various forms of cancer and metastatic diseases.

The compounds of the present invention are immunomodulatory agents which are especially indicated in the treatment of various skin graft and organ transplant reactions, neoplastic and metastatic diseases, and immune system diseases and disorders such as systemic lupus erythematosus and rheumatoid arthritis.

EP—A—0,030,632 designating AT, BE, CH, DE, FR, GB, IT, LI, NL and SE describes benzothiazole derivatives having the general formula

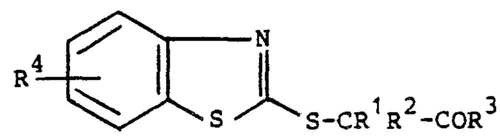

(A)

and their physiologically acceptable salts (wherein $R^1$ is hydrogen or methyl, $R^2$ is phenyl, tolyl, xylyl or pyridyl, $R^3$ is hydroxy, $C_1$—$C_4$ alkoxy or —$NHCH_2CH_2OH$ and $R^4$ is hydrogen, chlorine, bromine, hydroxy or $C_1$—$C_4$ alkoxy) as lipid level reducing agents.

EP—A—0,021,773 discloses compounds having the formula

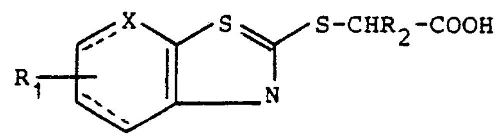

(B)

(wherein $R_1$ is hydrogen, halogen, amino, $C_1$—$C_6$ alkyl, $C_1$—$C_6$ alkoxy, trifluoromethyl or hydroxy, $R_2$ is *inter alia* phenyl or substituted phenyl, X is CH or N and the dotted lines represent optional double bonds in the indicated positions) as chemical intermediates.

The present invention is based upon the finding that a class of compounds that includes some compounds of formulae A and B are useful as pharmaceuticals, particularly as immunomodulatory agents.

According to one aspect the invention provides use of the following compounds for the manufacture of medicaments for use as immunomodulating agents. The compounds are those having the general formula:

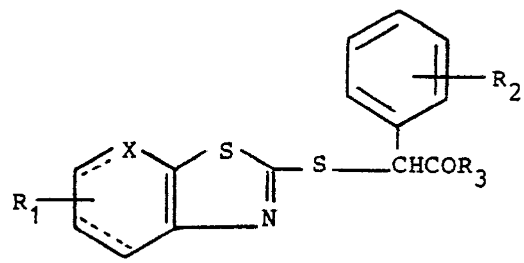

(I)

(wherein $R_1$ is hydrogen, halo, nitro, amino, lower alkyl, lower alkoxy, trifluoromethyl or hydroxy; $R_2$ is hydrogen, fluorine, chlorine, bromine, lower alkyl, lower alkoxy, amino, nitro or trifluoromethyl; $R_3$ is hydroxy, lower alkoxy, amino, hydroxy(lower)alkyl amino, N-(lower)alkanoylamino(lower)alkoxy or N-arylcarbamoyl(lower)alkylthio; X is CH or N, and where the dotted lines represent optional double bonds in the indicated positions, and the pharmacologically acceptable salts thereof. The compounds are new for use as a pharmaceutical except those having formula A. The compounds are new *per se* except those having formulae A and B.

A pharmaceutical composition may comprise a compound having the formula I or a pharmacologically acceptable salt thereof in association or combination with a pharmaceutically acceptable carrier. The said composition may be prepared by bringing the compound having formula I or a pharmacologically acceptable salt thereof into association or combination with a pharmaceutically acceptable carrier.

The term "lower alkyl" when used herein includes straight and branched chain hydrocarbon radicals having from 1 to about 6 carbon atoms. The terms "lower alkoxy" and "lower alkanoyl" in like manner designate radicals in which the hydrocarbon portion has 1 to about 6 carbon atoms.

The terms "halo" and "halide" when used herein refer to radicals of the elements fluorine, chlorine and bromine and chlorine and bromine, respectively.

The compounds having the formula I and their salts may be prepared by:—

(a) reacting a compound having the formula II

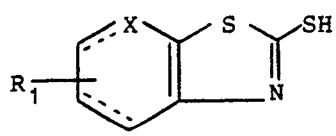

(II)

(wherein $R_1$, X and the dotted lines are as defined above) or a salt thereof with a compound having formula III

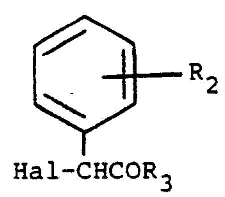

(III)

(wherein $R_2$ and $R_3$ are as defined above and Hal is chlorine or bromine) or a salt thereof; or

(b) reacting a compound having the formula IV

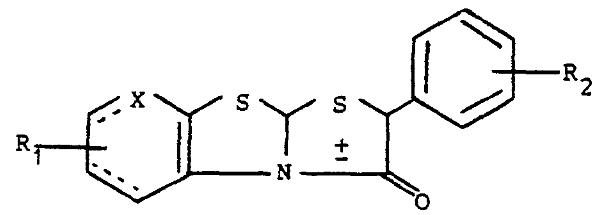

(IV)

(wherein $R_1$, $R_2$, X and the dotted lines are as defined above) with a nucleophilic reactant having the formula $R_3H$ (where $R_3$ is as defined above); and, if desired, a salt of a compound having formula I is converted into a compound having formula I or a compound having formula I is converted into a pharmacologically acceptable salt thereof.

Method (a) is preferably used where compounds having the formula I where $R_3$ is hydroxy and their salts are to be prepared, for instance, according to the following reaction sequence:

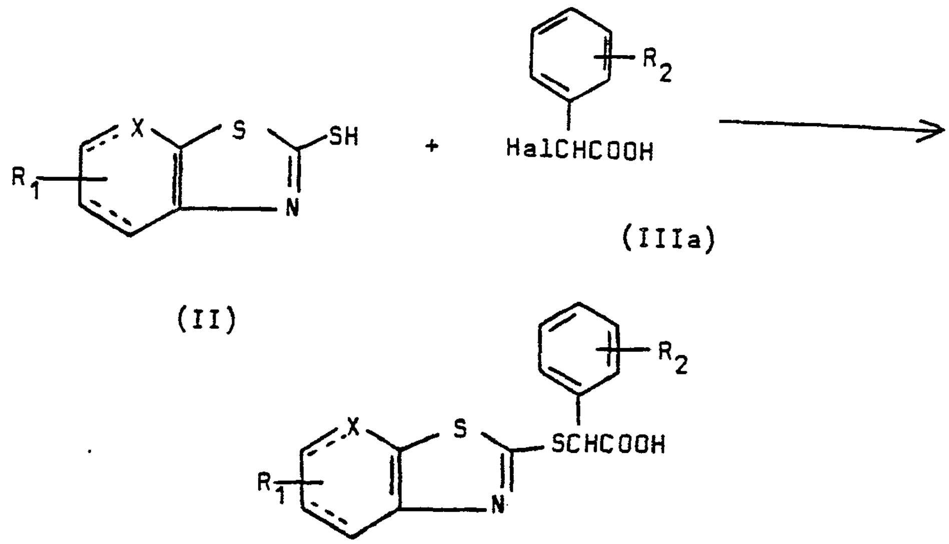

(IIa)

(II)

(Ia)

wherein $R_1$, $R_2$, X, Hal and the dotted lines are as defined hereinbefore. The reaction of the appropriately substituted 2-mercapto(aza)benzothiazole (II) and the α-halophenylacetic acid (IIIa) may be carried out in an organic solvent, such as for example acetone and can be performed at room temperature or at elevated temperatues. The substituted-2-mercapto(aza)benzothiazoles can be reacted in their alkali metal or alkaline earth metal salt form. The reaction can also be carried out in the presence of a scavenging agent for the liberated hydrohalide, such as a tertiary amine, for example triethylamine. This is preferred in the preparation of the azabenzothiazolylthioglycolic acids.

The derivatives of the α-phenyl-2-(aza)-benzothiazolylthioglycolic acids of the invention are most conveniently prepared by reacting the tricyclic mesionic didehydro compounds having formula IV with appropriate nucleophilic reactants. This reaction, involving the ring cleavage of the terminal thiazole ring, is as follows:

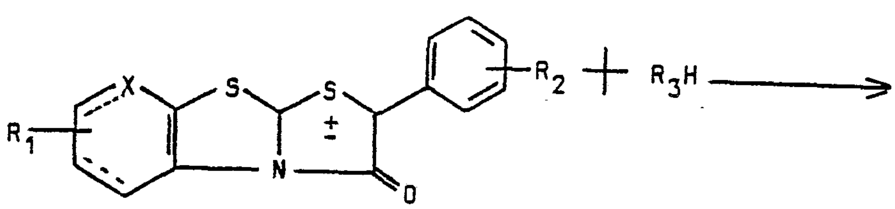

(IV)

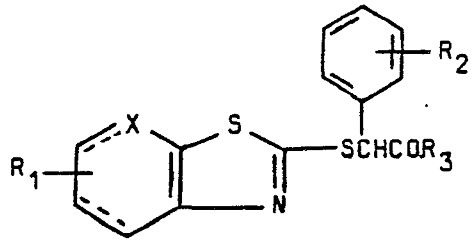

(I)

wherein $R_1$, $R_2$, $R_3$, and X are as described hereinbefore. The reaction is carried out in a suitable organic solvent, such as for example methylene chloride and over a range of temperatures, such as room temperature as well as under reflux conditions, depending on the nature of the nucleophilic reactant being used.

The compounds of formula I may be prepared in the form of acid addition salts, particularly the hydrohalide salt where method (a) is carried out. Such salts may be converted into the compounds having formula I by removing the acid in known manner. The compounds of formula (I) in which $R_3$ is hydroxy may be prepared in the form of the carboxylic acid and the carboxylate salts. The acid and salts are interconvertible in known manner, for instance, neutralisation of the acid with a base yields a salt or addition of an acid to the salt yields the carboxylic acid.

The starting materials having formulae II, III and IV and the nucleophilic reagent having the formula $R_3H$ are generally known and, where new, can be prepared in known manner. The preparation of compounds having the formula IV is described in our EP—B—21773.

The compounds of the invention are immunomodulators. The compounds have therapeutic application in a variety of situations in which immunomodulation is indicated. Thus, the compounds are useful in treating allograft reactions, organ transplant reactions, and graft vs. host reactions. The compounds permit the host to accept the graft without destroying the host's immunity to other infections. The compounds are also useful in the treatment of autoimmune diseases, such as systemic lupus erythematosus (SLE). Further, the compounds of the invention inhibit the production of the immunoglobulins, which are so pathologic to autoimmune disease such as SLE, as well as the production of antigen-antibody complexes which are the causative agents of renal and inflammatory processes in arthritis and autoimmune diseases. Thus, the compounds of the invention are also useful in the treatment of such conditions as rheumatoid arthritis. Moreover, the compounds can be used in the treatment of neoplastic diseases, as well as metastatic diseases.

Accordingly, the invention is further directed to a method of modulating the immune response in warm-blooded animals in need of modulation of the immune response by administering to a warm-blooded animal an amount effective to bring about said modulation of the immune response of a compound having the general formula I.

When the compounds of the invention are employed as immunomodulators, they can be formulated into oral dosage forms such as tablets, capsules and the like. The compounds can be administered alone or by combining them with conventional carriers, such as magnesium carbonate, magnesium stearate, talc, sugar, lactose, pectin, dextrin, starch, gelatin, tragacanth, methyl cellulose, sodium carboxymethyl cellulose, low melting wax, cocoa butter, and the like. Diluents, flavouring agents, solubilisers, lubricants, suspending agents, binders, tablet-disintegrating agents and the like may be employed. The compounds may be encapsulated with or without other carriers. In all cases the proportion of active ingredients in said compositions both solid and liquid will be at least sufficient to impart immunomodulatory activity thereto on oral administration. The compounds may also be injected parenterally, in which case they are used in the form of a sterile solution containing other solutes, for example, enough saline or glucose to make the solution isotonic.

The dosage requirements vary with the particular compositions employed, the route of administration, the severity of the symptoms presented and the particular subject being treated. Treatment will generally be initiated with small dosages less than the optimum dose of the compound. Thereafter the dosage is increased until the optimum effect under the circumstances is reached. In general, the compounds of the invention are most desirably administered at a concentration level that will generally afford effective results without causing any harmful or deleterious side effects. With large animals (about 70 kg. body weight), for injection administration the dose may be from about 25 milligrams to about 50 milligrams and for oral administration the dose may be from about 50 milligrams to about 200 milligrams and preferably from about 50 milligrams to about 100 milligrams per day either as a single unit dose, or if desired, the dosage may be divided into convenient subunits administered at convenient times throughout the day.

The immunomodulatory effect of the compounds of the invention may be demonstrated by standard pharmacological and histological procedures. The pharmacological procedures are described more fully in Examples 17 and 18 given hereinafter.

These procedures illustrate the ability of the compounds of the invention to exert an

immunomodulatory effect, especially selectively on the cell-mediated immunity by directly measuring the effect of the compounds on the humoral and cell-mediated immunity, and include evaluation of the compounds in the Plescia anti-SRBC antibody production assay and evaluation in the mitogen activated murine T lymphocyte proliferation test.

Examples 1 to 5 and 7 to 16 illustrate the preparation of compounds having formula I and Example 6 illustrates the preparation of an intermediate compound having formula IV and being known from EP—A—21773.

Example 1

α-(2-Benzothiazolylthio)benzeneacetic acid

A. α-(2-Benzothiazolylthio)benzene acetic acid, hydrobromide

A solution of 16.7 g (0.10 m) 2-mercaptobenzothiazole and 21.5 g (0.10 m) α-bromo-α-phenylacetic acid in acetone is heated to reflux for 5 hours and the solvent is then removed. The residual oil is triturated in acetone and 31 g of solid collected. The compound melts at 192—4°C.

*Analysis:* $C_{15}H_{11}NO_2S_2 \cdot HBr$

| | | | | | |
|---|---|---|---|---|---|
| Calculated: | C, 47.12; | H, 3.17; | N, 3.66; | Br, 20.90; | S, 16.77 |
| Found: | C, 47.22; | H, 3.06; | N, 3.69; | Br, 20.93; | S, 16.73. |

B. α-(2-Benzothiazolylthio)benzene acetic acid

The hydrobromide salt of A above is stirred in water at room temperature overnight, the solid collected and air dried. The crude material is recrystallised from acetonitrile to give the title compound in 70% yield and melting at 152—4°C.

*Analysis for:* $C_{15}H_{11}NO_2S_2$

| | | | |
|---|---|---|---|
| Calculated: | C, 59.78; | H, 3.68; | N, 4.65 |
| Found: | C, 59.81; | H, 3.85; | N, 4.77. |

Example 2

α-(5-Chlorobenzothiazol-2-ylthio)benzene acetic acid

50.0 g (0.248 m) 5-chloro-2-mercaptobenzothiazole and 53.0 g (0.248 m) α-bromophenylacetic acid are dissolved in 1.5 l acetone and the solution is heated for 4 hours in the presence of 50 ml glacial acetic acid. The solution is concentrated to a smaller volume (about 200 ml) and the residual solid (90 g) is collected. The resulting salt is suspended in 1 l of water and the mixture is stirred at room temperature overnight. The collected solid is recrystallised from 2.5 l acetonitrile to give a total of 64 g (85% yield) of title compound melting at 190—2°C.

*Analysis for:* $C_{15}H_{10}ClNO_2S_2$

| | | | |
|---|---|---|---|
| Calculated: | C, 53.64; | H, 3.00; | N, 4.17 |
| Found: | C, 53.83; | H, 3.13; | N, 4.13. |

Example 3

Sodium salt of α-(5-chlorobenzothiazol-2-ylthio)benzene acetic acid

To a solution of 10 g (0.03 m) of the compound of Example 2 in 600 ml of acetone is added an alcoholic NaOH (1.2 g (0.03 m) in 20 ml ethanol) solution. The precipitate is collected and recrystallised from water. 8.0 g (71% yield) of title compound is isolated as a monohydrate melting at 238—42°C (dec.).

*Analysis for:* $C_{15}H_9ClNO_2S_2Na\ H_2O$

| | | | |
|---|---|---|---|
| Calculated: | C, 47.93; | H, 2.95; | N, 3.73 |
| Found: | C, 47.7; | H, 3.12; | N, 3.79. |

Example 4

α-(5-chlorobenzothiazol-2-ylthio)-α-(*p*-chlorophenyl)acetic acid

*Method A.* An acetone solution of 12.1 g (0.06 m) 5-chloro-2-mercaptobenzothiazole and 15.0 g (0.06 m) α-bromo-α-(p-chlorophenyl)-acetic acid is heated in the presence of 30 ml glacial acetic acid for 5 hours. After solvent removal, the residual solid is collected. This solid (9 g of the hydrobromic salt of the title compound) is stirred in water overnight and the solid is collected and air dried. The crude material is recrystallised from acetonitrile to give 4.7 g (21% yield) of the title compound melting at 157—8°C.

*Method B.* An acetone solution of the potassium salt of 5-chloro-2-mercaptobenzothiazole (0.05 m) and α-bromo-α-(*p*-chlorophenyl)-acetic acid (0.05 m) are heated to reflux for 3 days. The solid (KBr), formed during the reaction, is filtered off and the filtrate is concentrated, and the residual solid triturated with a mixture of acetone and hexane. The crude material is recrystallised from benzene to give 7.7 g (42% yield) of title compound melting at 157—8°C.

*Method C.* A methylene chloride solution of 10.0 g (0.05 m) 5-chloro-2-mercaptobenzothiazole, 12.5 g (0.05 m) α-bromo-α-(*p*-chlorophenyl)-acetic acid and 10.0 g (0.10 m) triethylamine is heated to reflux

overnight. The reaction mixture is washed first with a dilute hydrochloric acid solution, then with water and finally dried over $MgSO_4$. After the methylene chloride is removed, the residual solid is triturated with acetonitrile to give 13.6 g (74% yield) of title compound melting at 157—8°C.

*Analysis for:* $C_{15}H_9Cl_2NO_2S_2$
Calculated: C, 48.65; H, 2.45; N, 3.78
Found: C, 48.69; H, 2.58; N, 3.79.

Example 5

α-(p-Chlorophenyl)-α-(benzothiazol-2-yl-thio) acetic acid

Following the procedure of Example 4, Method C and substituting 2-mercaptobenzothiazole for 5-chloro-2-mercaptobenzothiazole, the title compound is prepared in 34% yield and having a melting point of 185—7°C (dec.)

*Analysis for:* $C_{15}H_{10}ClNO_2S_2$
Calculated: C, 53.64; H, 3.00; N, 4.17
Found: C, 54.07; H, 3.17; N, 4.13.

Example 6

Preparation of 6-Chloro-2-phenylthiazolo[2,3-b]benzothiazol-3(2*H*)-one mesionic didehydro derivative

31 g (0.092 m) of the compound of Example 2 is suspended in 3.5 l methylene chloride and the mixture is heated to gentle reflux in the presence of 25 ml acetic anhydride. The solid is gradually dissolved, the solution turning reddish. After heating overnight, the solution is filtered and concentrated to 200 ml. The residual orange solid, weighing 29.5 g (quantitative yield), is collected and has a melting point of 215—6°C.

Example 7

α-(5-Chlorobenzothiazol-2-ylthio)benzene acetic acid

7 g of the compound of Example 6 is dissolved in 100 ml N,N-dimethylacetamide and the solution is allowed to stand at room temperature for 2 days in the presence of 5 ml water. The solution is diluted with 120 ml water and the resulting solid is collected and air dried. The crude material is recrystallised from 500 ml benzene. 5.9 g (80% yield) of purified title compound is obtained, having a melting point of 190—1°C.

*Analysis for:* $C_{15}H_{10}ClNO_2S_2$
Calculated: C, 53.64; H, 3.00; N, 4.17
Found: C, 53.83; H, 3.13; N, 4.13.

Example 8

α-(5-Chloro-2-benzothiazolylthio)benzene acetic acid, ethyl ester

8.0 g of the compound of Example 6 is dissolved in ethanol and the solution is warmed over a steam bath until the orange colour is discharged. The solvent is removed and oily residue is recrystallised from hexane. 6.6 g (73% yield) of title compound is obtained, having a melting point of 62—4°C.

*Analysis for:* $C_{17}H_{14}ClNO_2S_2$
Calculated: C, 56.11; H, 3.88; N, 3.85
Found: C, 56.05; H, 3.92; N, 3.84.

Example 9

α-(5-Chlorobenzothiazol-2-ylthio)-α-phenylacetamide

To 6.0 g (0.189 m) of the compound of Example 6 in 300 ml methylene chloride is introduced gaseous ammonia. The orange solid dissolves with discharge of the orange coloration by the precipitation of a white solid. After 15 minutes, the introduction of ammonia is stopped and the mixture is stirred for 30 minutes. The solid is collected and a further quantity of solid is recovered upon removal of the solvent from the filtrate. The combined solids are recrystallised from acetonitrile to give 5.0 g (79% yield) of title compound having a melting point of 181—3°C.

*Analysis for:* $C_{15}H_{11}ClN_2OS_2$
Calculated: C, 53.80; H, 3.31; N, 8.37
Found: C, 53.67; H, 3.41; N, 8.44.

Example 10

α-[(5-Chloro-2-benzothiazolyl)thio]-N-(2-hydroxyethyl)benzene acetamide

6.2 g (0.02 m) of the compound of Example 6 and 1.2 g (0.02 m) ethanolamine in a methylene chloride solution are warmed over a steam bath until dissolution is complete and the colouration discharged. The solution is filtered and the solvent removed. The residue is triturated with ether, the solid is collected and

dried in a drying oven. 6.2 g (82% yield) of title compound, having a melting point of 148—9°C, is obtained.

*Analysis for:* $C_{17}H_{15}ClN_2O_2S_2$
Calculated: C, 53.88; H, 3.99; N, 7.40
Found: C, 54.03; H, 4.14; N, 7.46.

Example 11

α-[(5-Chloro-2-benzothiazolyl)thio]benzene acetic acid, [2-(acetylamino)ethyl]ester

3.18 g (0.01 m) of the compound of Example 6 and 1.03 g (0.01 m) N-acetylethanolamine are dissolved in methylene chloride and the solution is heated to reflux for 3 days. After removal of the solvent, the oily residue is recrystallised from 100 ml ether. 3.5 g (83% yield) of title compound, having a melting point of 107—8°C, is obtained.

*Analysis for:* $C_{19}H_{17}ClN_2O_3S_2$
Calculated: C, 54.21; H, 4.07; N, 6.66
Found: C, 53.95; H, 4.14; N, 6.59.

Example 12

α-[(5-Chloro-2-benzothiazolyl)thio]benzeneethanethioic acid, S-[2-(2-naphthalenylamino)-2-oxoethyl]ester

3.18 g (0.01 m) of the compound of Example 6 and 2.17 g (0.01 m) α-mercapto-N-(2-naphthyl) acetamide are dissolved in methylene chloride and the mixture is heated to reflux for 7 days. The progress of the reaction is monitored by TLC. After removal of solvent, the residue is recrystallised from ether. 2.3 g (42% yield) of title compound, having a melting point of 144—5°C, is obtained.

*Analysis for:* $C_{27}H_{19}ClN_2O_2S_3$
Calculated: C, 60.60; H, 3.58; N, 5.24
Found: C, 60.89; H, 3.78; N, 5.27.

Example 13

α-[(5-Chlorothiazolo[5,4-*b*]pyridin-2-yl)thio]-α-phenyl acetic acid

A methylene chloride solution of 4.05 g (0.02 m) 5-chloro-2-mercaptothiazolo[5,4-*b*]pyridine, 4.30 g (0.02 m) α-bromophenylacetic acid and 4.0 g (0.04 m) triethylamine is heated to gentle reflux overnight. The methylene chloride solution is extracted twice with a dilute hydrochloric acid solution and once with water and then dried over anhydrous $MgSO_4$. After the solvent is removed, the residual solid is recrystallised from acetonitrile. 5.0 g (74% yield) of title compound, melting at 175—7°C, is obtained.

*Analysis for:* $C_{14}H_9ClN_2O_2S_2$
Calculated: C, 49.92; H, 2.69; N, 8.32
Found: C, 49.97; H, 2.85; N, 8.37.

Example 14

α-[(5-Chlorothiazolo[5,4-*b*]pyridin-2-yl)thio]-α-(p-chlorophenyl) acetic acid

A methylene chloride solution of 6.0 g (0.03 m) of 5-chloro-2-mercaptothiazolo[5,4-*b*]pyridine, 7.5 g (0.03 m) α-bromo-α-(*p*-chlorophenyl) acetic acid and 6.0 g (0.06 m) triethylamine is heated to gentle reflux overnight. The solution is extracted twice with a dilute hydrochloric acid solution and then once with water. After drying over anhydrous $MgSO_4$, the solvent is removed and the residual solid is recrystallised from acetonitrile. 7.5 g (68% yield) of title compound, melting at 144—6°C, is obtained.

*Analysis for:* $C_{14}H_8Cl_2N_2O_2S_2$
Calculated: C, 45.29; H, 2.17; N, 7.55
Found: C, 44.87; H, 2.27; N, 7.59.

Example 15

α-Phenyl-α-[(4,5,6,7-tetrahydrobenzothiazol-2-yl)thio] acetic acid

A methylene chloride solution of 5.13 g (0.03 m) 2-mercapto-4,5,6,7-tetrahydrobenzothiazole, α-bromophenylacetic acid and 6.0 g (0.06 m) triethylamine is heated to gentle reflux overnight. The solution is extracted twice with a dilute hydrochloric acid solution, once with water and is then dried over anhydrous $MgSO_4$. The residual solid after solvent removal is recrystallised from acetonitrile. 7.0 g (77% yield) of title compound, melting at 118—20°C, is obtained.

*Analysis for:* $C_{15}H_{15}NO_2S_2$
Calculated: C, 58.99; N, 4.95; N, 4.59
Found: C, 59.00; H, 4.94; N, 4.52.

Example 16

α-[(6-Nitro-2-benzothiazolyl)thio]benzeneacetic acid

A methylene chloride solution of 5.0 g (0.0235 m) 2-mercapto-6-nitrobenzothiazole, 5.2 g (0.235 m) α-bromophenylacetic acid and 5.0 g (0.05 m) triethylamine is heated to gentle reflux overnight. The solution is washed twice with a dilute hydrochloric acid solution and once with water. The solution is dried over anhydrous $MgSO_4$ and then concentrated. The residue is recrystallised once from benzene and then from acetonitrile. 1.5 g (18% yield) of title compound, having a melting point of 152—5°C, is obtained.

| | | | |
|---|---|---|---|
| *Analysis for:* $C_{15}H_{10}N_2O_4S_2$ | | | |
| Calculated: | C, 52.01; | H, 2.91; | N, 8.09 |
| Found: | C, 51.72; | H, 2.97; | N, 8.01. |

Example 17

The Plescia anti-SRBC (sheep red blood cells) antibody induction assay is a very sensitive test which is used to measure the ability of an immunomodulatory agent to augment or depress SRBC-induced production of SRBC-specific IgM and IgG antibodies. That is, this assay provides specific information regarding the ability of an immunomodulatory agent to induce the entire immune system to provide an enhanced or depressed antibody response to challenge with an antigen, which is the assay in SRBC. The effect of potential immunomodulatory compounds on T cells and their various subpopulations, as well as on B cells, can be studied in this assay.

The total assay procedure is essentially in three parts. In the first part, antigen-primed splenocytes (spleen cells) are prepared. C57B1/6J male mice are injected intraperitoneally with $10^7$SRBC (in pyrogen-free isotonic saline). Ten days later, the animals are sacrificed, their spleens excised and a suspensions of spleen cells is prepared therefrom. The suspension is prepared using 1 ml of tissue culture medium (Click medium supplemented with 10% fetal calf serum) per spleen and all suspensions are pooled into a single suspension. The number of viable nucleated spleen cells in the suspension is counted and adjusted to $10^7$/ml.

In the second part, 1 ml of the above suspension of prime spleen cells is added to sterile culture tubes to which is added 1 ml of a standard suspension of SRBC ($10^7$/ml of tissue culture medium). Stock solutions of compounds to be tested are prepared in tissue culture medium and from this appropriate dilutions are made to cover a range of concentrations. To each culture tube is added 0.1 ml of each compound dilution, while 0.1 ml of medium alone is added to the control tubes. The tubes are capped, mixed and then placed in a humidified −5% $CO_2$ incubator at 37°C for 6 days. The tubes are then centrifuged at 1000×g for about 10 minutes to sediment the cells and the supernatants decanted into test tubes.

In the third part, 2-fold serial dilutions of the supernatants, from 1/1 to 1/32, are made and 0.1 ml of each dilution is added to individual test tubes. To each tube there is added 0.1 ml of SRBC ($10^9$/ml), the tubes are mixed and allowed to stand at room temperature for 15—30 minutes. Then 0.1 ml complement (guinea pig serum diluted to contain a slight excess of complement) is added to each tube, mixed and the tubes are placed in a 37°C water bath for lysis of SRBC to occur. Only SRBC that have been sensitised by anti-SRBC antibody in the supernatant will lyse. 2.0 ml of isotonic saline is added to each tube, mixed and centrifuged to sediment unlysed cells and to recover the supernatant containing hemoglobin from the lysed SRBC. The supernatants' absorbance is measured at 541 nm.

Since the per cent of SRBC that is lysed is proportional to the amount of anti-SRBC antibody present in the test cell culture supernatant, and since lysis is measured in terms of the hemoglobin that is released from the lysed SRBC, it is the absorbance of the released hemoglobin that is measured in order to determine the quantity of anti-SRBC antibody present in the supernatant. Because the assay is most precise at the range of 50% lysis of SRBC in the presence of excess complement, the volume of supernatant that produces 50% lysis of SRBC, by definition, contains 1 unit of antibody called a 50% hemolytic unit (H50). One divided by this volume gives the number of H50's per ml of test supernatant. A standard curve of absorbance vs. H50/ml for control supernatant is then prepared, and the concentration of antibody in other test culture supernatants, for each dilution, is determined. The content of anti-SRBC antibody for the various test compounds at their various concentrations show whether the compound has caused stimulation or suppression of the immune response.

The results of this assay for several compounds of the invention are tabulated in Table 1, where the data is presented as H50 units of antibody/ml and % change from control.

TABLE 1

Experiment 1

| Compound (of Example No) | Quantity µg/ml | Units of Antibody | % Change from Control |
|---|---|---|---|
| Example 2 | 25 | 30.32 | −428 |
| | 2.5 | 84.96 | −147 |
| Example 4 | 25 | 62.3 | −200 |
| | 2.5 | 124.6 | 0 |
| Control | — | 124.9 | — |

Experiment 2

| Compound (of Example No) | Quantity µg/ml | Units of Antibody | % Change from Control |
|---|---|---|---|
| Example 4 | 25 | 47.53 | −348 |
| Example 3 | 25 | 48.26 | −343 |
| Control | — | 165.4 | — |

The results show that the compounds of the invention have a profound and significant depressive effect on the immune response, an effect which is further substantiated by the results in the mitogen activated murine T lymphocyte proliferation test.

Example 18

The mitogen activated murine T lymphocyte proliferation test is used to determine the effects of immunomodulatory agents on the proliferation of murine T cell populations. The test is run to determine whether test compounds will enhance or depress the proliferation of T cells, whose proliferation has been initially stimulated by a mitogen, such as Concanavallin A (Con A). To exclude the possibility that the test compounds act at Con A binding sites, the T cell proliferation can be studied in a system in which the test compounds are incubated with the T cells prior to the addition Con A instead of in a system in which test compounds and Con A are added to the cell cultures concomitantly.

The test procedure is as follows:

T lymphocytes are isolated from spleens of male CBA/J or NZB mice. Cell homogenates are prepared in Hank's balanced salt solution (HBSS). After removal of larger particles and repeated washing of the cells in HBSS they are suspended in minimum essential medium (MEM) and passed through a glass wool column to remove macrophages. The cells are then incubated on a nylon wool column at 37°C, 95% air, 5% $CO_2$, for 45 minutes. The non-adherent T lymphocytes are then eluted from the column, counted, and adjusted to $20 \times 10^6$ cells/ml. 50 µl of cells are cultured (37°C, 95% air, 5% air $CO_2$) with compound, or compound and mitogen (0.025 µg/culture of Concanavalin A) for 48 hours before the addition of 0.5 µCi of $^3H$-thymidine for the last 16 hours of culture. The total volume of the culture system is 200 µl. The cells are then harvested on a multiple automatic sample harvester (Mash II), the glass fibre discs placed in 10 ml of xylene base scintillation fluid, and counted for 1 minute in a liquid scintillation counter.

The incubation experiments are conducted in two ways:

1) lymphocytes are incubated for 2 hours with test compounds, washed and cultured with different concentrations of Con A; and

2) lymphocytes are incubated for 24 hours with varied amounts of Con A, washed and then cultured with test compounds. The concentration of Con A used is either 0.04 or 0.05 µg/culture. The effects of some compounds of the invention are summarised as follows:

A). Sodium salt of α-(5-chlorobenzothiazol-2-ylthio)benzene acetic acid. When the compound is cultured concomitantly with Con A present in the amounts of 0.02; 0.04; 0.06; and 0.08 µg/culture, the compound inhibits the Con A induced blastogenesis at concentrations of 0.1, 1.0, 5 and 10 µg/culture. When the compound is cultured with lymphocytes prior to addition of 0.04 or 0.05 µg/culture of Con A, the compound failed to show inhibitory activity. When the compound is cultured with lymphocytes 24 hours after the cells are incubated with 0.04 or 0.05 µg/culture of Con A, at concentrations of >2 or 3 µg/culture the compound showed strong inhibition of lymphocyte blastogenesis.

B). α-(5-Chlorobenzothiazol-2-ylthio)-α-(*p*-chlorophenyl) acetic acid. When this compound is cultured concomitantly with Con A present in the amounts of 0.02; 0.03; 0.04 and 0/06 µg/culture, the compound

inhibits the Con A induced blastogenesis at concentrations >2 µg/culture. When the compound is cultured with lymphocytes prior to addition of 0.04 or 0.05 µg/culture of Con A, the compound shows consistent inhibitory activity at 3 and 5 µg/culture. When the compound is cultured with lymphocytes 24 hours after the cells are incubated with 0.04 or 0.05 µg/culture of Con A, at concentrations of >2 or 3 µg/culture the compound showed strong inhibition of blastogenesis.

These results clearly demonstrate that the compounds of the invention are strong and consistent inhibitors of T cell proliferation, which has been mitogenically induced. It is also clear that the compounds are able to perturb T lymphocyte blastogenesis when Con A binding sites are occupied, and so the compounds are not in competition with Con A for cell binding sites.

**Description for the Contracting State: LU**

The invention relates to α-phenyl-2-(aza)benzothiazolylthioglycolic acids, derivatives thereof and pharmacologically acceptable salts thereof. The compounds are useful as modulators of the immune response.

In recent years, the rapid upsurge in immunological research has brought about a greater appreciation and understanding of the complexities of the immune response. While the traditional overall view of the immune system remains, new discoveries have radically changed some thinking about the details of the system. Thus, the immune system is still divided into humoral immunity, populated with B cells and responsible for antibody formation, and cell-mediated immunity, populated with T cells and responsible for the rejection of organ transplants or skin grafts, as well as the defence mechanism against various foreign biological matter and endogenous neoplastic growths.

It is only in the last decade or so, however, that the concept has been accepted that different cell populations interact in the induction and expression of both humoral and cell-mediated immunity. Thus, subpopulations of B cells and T cells have been described, such as for example "suppressor and "helper" T cells. In a number of animal models, it has been postulated that the helper T cells act in the induction of a complete antibody response to many antigens, whereas T suppressor cells are capable of preventing or terminating such responses. It is now believed that positive and negative cellular interactions control the ultimate degree of immune response. So, it is believed that any given immune response is regulated, and that the degree and mode of regulation may ultimately explain the various reactions, diseases, and disorders which are the manifestations of the operation of the organism's immune system.

The T cell subpopulations of suppressor and helper T cells have been implicated in a number of immune response manifestations. Thus, the loss of suppressor T cells is now believed to be a major factor in such autoimmune connective tissue disease as systemic lupus erythematosus. Moreover, in the latter case, as well as in probable impaired immune system responses such as rheumatoid arthritis, it is believed the helper T cells exacerbate the condition.

Also, the theory has been advanced that T suppressor cell hypofunctioning, resulting in inadequate T—B cell cooperation in the immune response, with continuous B cell stimulation and subsequent antibody production may be the cause of the production of antigen-antibody complexes which are the causative agents of renal and inflammatory processes in arthritis and autoimmune diseases.

Thus, it is now apparent that a number of lymphopoietic disorders are undoubtedly associated with abnormalities of T cell and especially suppressor cell function. The loss of suppressor function is at least an early event in certain immune response diseases and is a disease-perpetuating mechanism in other. The loss of suppressor function probably leads to excessive lymphoid cell proliferation and may significantly contribute to lymphoproliferative disorders. The conditions created thereby may be exacerbated by helper T cells.

The role of immunomodulatory agents in the treatment of immune diseases and disorders, as well as in the attempt to prolong the life of organ transplants and skin grafts, has been to suppress the immune response, especially of cell-mediated immunity. By suppressing the cell-mediated immune response, it is possible to delay and possibly prevent the host organism from rejecting a skin graft or organ transplant, or the graft from immunologically rejecting the host (graft vs. host reaction). Similarly, enhancing or reinstituting suppressor function by immunomodulator therapy is a beneficial course of treatment for autoimmune and probable autoimmune diseases and disorders. Immunotherapy is also being recognised as an important aspect of the treatment, especially chemotherapeutic, of various forms of cancer and metastatic diseases.

The compounds of the present invention are immunomodulatory agents which are especially indicated in the treatment of various skin graft and organ transplant reactions, neoplastic and metastatic diseases, and immune system diseases and disorders such as systemic lupus erythematosus and rheumatoid arthritis.

EP—A—0,021,773 discloses compounds having the formula

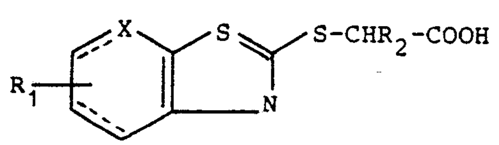

(B)

(wherein $R_1$ is hydrogen, halogen, amino, $C_1$—$C_6$ alkyl, $C_1$—$C_6$ alkoxy, trifluoromethyl or hydroxy, $R_2$ is *inter alia* phenyl or substituted phenyl, X is CH or N and the dotted lines represent optional double bonds in the indicated positions) as chemical intermediates.

The present invention is based upon the finding that a class of compounds that includes some compounds of formula B are useful as pharmaceuticals, particularly as immunomodulatory agents.

According to one aspect of the invention provides compounds for use as pharmaceuticals particularly for use as immunomodulating agents. The compounds are those having the general formula:

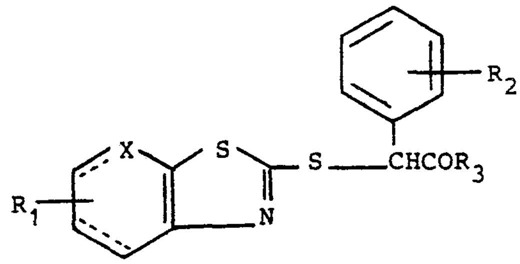

(I)

wherein $R_1$ is hydrogen, halo, nitro, amino, lower alkyl, lower alkoxy, trifluoromethyl or hydroxy; $R_2$ is hydrogen, fluorine, chlorine, bromine, lower alkyl, lower alkoxy, amino, nitro or trifluoromethyl; $R_3$ is hydroxy, lower alkoxy, amino, hydroxy(lower)alkyl amino, N-(lower)alkanoylamino(lower)alkoxy or N-arylcarbamoyl(lower)alkylthio; X is CH or N, and where the dotted lines represent optional double bonds in the indicated positions, and the pharmacologically acceptable salts thereof. The compounds are new *per se* subject to the proviso that when $R_3$ is hydroxy then $R_1$ is nitro.

According to a second aspect, the invention provides a pharmaceutical composition comprising a compound having the formula I or a pharmacologically acceptable salt thereof in association or combination with a pharmaceutically acceptable carrier. The invention also includes a method of preparing the said composition by bringing the compound having formula I or a pharmacologically acceptable salt thereof into association or combination with a pharmaceutically acceptable carrier.

The term "lower alkyl" when used herein includes straight and branched chain hydrocarbon radicals having from 1 to about 6 carbon atoms. The terms "lower alkoxy" and "lower alkanoyl" in like manner designate radicals in which the hydrocarbon portion has 1 to about 6 carbon atoms.

The terms "halo" and "halide" when used herein refer to radicals of the elements fluorine, chlorine and bromine and chlorine and bromine, respectively.

The compounds having the formula I and their salts may be prepared by:—

(a) reacting a compound having the formula II

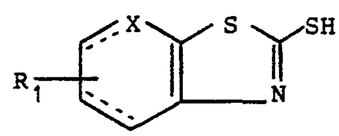

(II)

(wherein $R_1$, X and the dotted lines are as defined above) or a salt thereof with a compound having formula III

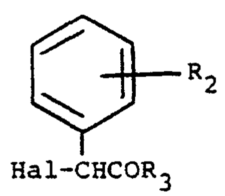

(III)

(wherein $R_2$ and $R_3$ are as defined above and Hal is chlorine or bromine) or a salt thereof; or

(b) reacting a compound having the formula IV

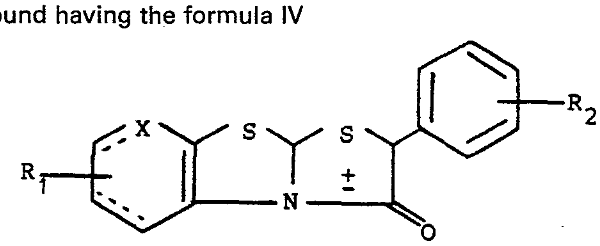

(IV)

(wherein $R_1$, $R_2$, X and the dotted lines are as defined above) with a nucleophilic reactant having the formula $R_3H$ (where $R_3$ is as defined above); and, if desired, a salt of a compound having formula I is

converted into a compound having formula I or a compound having formula I is converted into a pharmacologically acceptable salt thereof.

Method (a) is preferably used where compounds having the formula I where $R_3$ is hydroxy and their salts are to be prepared, for instance, according to the following reaction sequence:

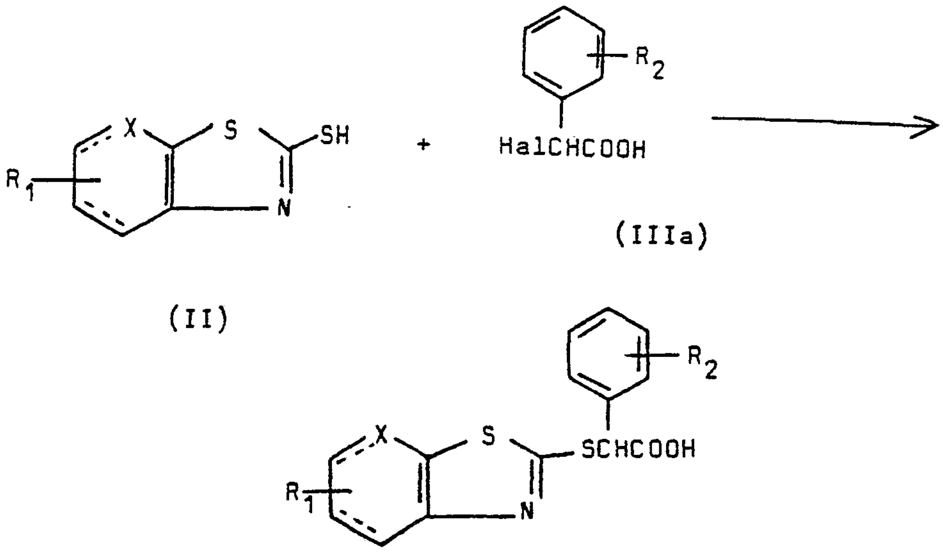

(II)

(IIIa)

(Ia)

wherein $R_1$, $R_2$, X, Hal and the dotted lines are as defined hereinbefore. The reaction of the appropriately substituted 2-mercapto(aza)benzothiazole (II) and the α-halophenylacetic acid (IIIa) may be carried out in an organic solvent, such as for example acetone and can be performed at room temperature or at elevated temperatures. The substituted-2-mercapto(aza)benzothiazoles can be reacted in their alkali metal or alkaline earth metal salt form. The reaction can also be carried out in the presence of a scavenging agent for the liberated hydrohalide, such as a tertiary amine, for example triethylamine. This is preferred in the preparation of the azabenzothiazolylthioglycolic acids.

The derivatives of the α-phenyl-2-(aza)-benzothiazolylthioglycolic acids of the invention are most conveniently prepared by reacting the tricyclic mesionic didehydro compounds having formula IV with appropriate nucleophilic reactants. This reaction, involving the ring cleavage of the terminal thiazole ring, is as follows:

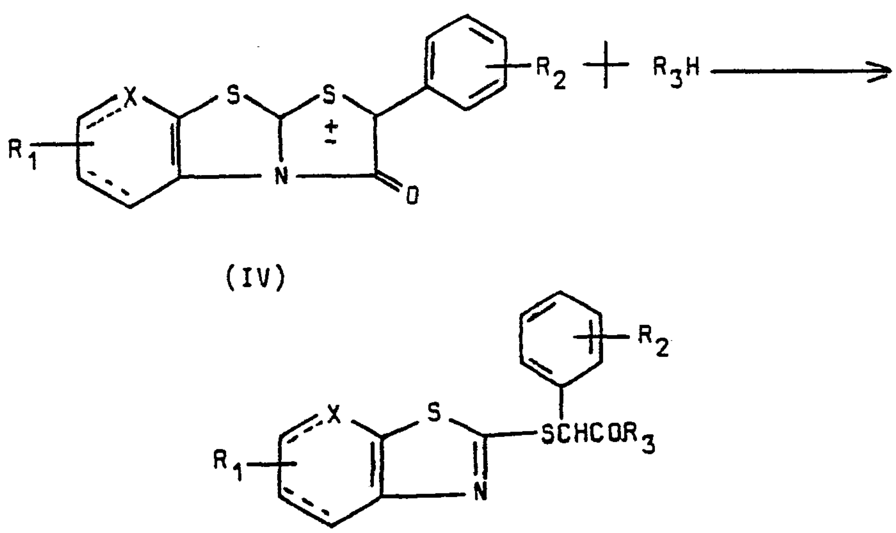

(IV)

(I)

wherein $R_1$, $R_2$, $R_3$, and X are as described hereinbefore. The reaction is carried out in a suitable organic solvent, such as for example methylene chloride and over a range of temperatures, such as room temperature as well as under reflux conditions, depending on the nature of the nucleophilic reactant being used.

The compounds of formula I may be prepared in the form of acid addition salts, particularly the hydrohalide salt where method (a) is carried out. Such salts may be converted into the compounds having formula I by removing the acid in known manner. The compounds of formula in which $R_3$ is hydroxy may be prepared in the form of the carboxylic acid and the carboxylate salts. The acid and salts are interconvertible in known manner, for instance, neutralisation of the acid with a base yields a salt or addition of an acid to the salt yields the carboxylic acid.

The starting materials having formulae II, III and IV and the nucleophilic reagent having the formula $R_3H$ are generally known and, where new, can be prepared in known manner. The preparation of compounds having the formula IV is described in our EP—B—21773.

The compounds of the invention are immunomodulators. The compounds have therapeutic application in a variety of situations in which immunomodulation is indicated. Thus, the compounds are useful in treating allograft reactions, organ transplant reactions, and graft vs host reactions. The compounds permit the host to accept the graft without destroying the host's immunity to other infections. The compounds are also useful in the treatment of autoimmune diseases, such as systemic lupus erythematosus (SLE). Further, the compounds of the invention inhibit the production of the immunoglobulins, which are so pathologic to autoimmune disease such as SLE, as well as the production of antigen-antibody complexes which are the causative agents of renal and inflammatory processes in arthritis and autoimmune diseases. Thus, the compounds of the invention are also useful in the treatment of such conditions as rheumatoid arthritis. Moreover, the compounds can be used in the treatment of neoplastic diseases, as well as metastatic diseases.

Accordingly, the invention is further directed to a method of modulating the immune response in warm-blooded animals in need of modulation of the immune response by administering to a warm-blooded animal an amount effective to bring about said modulation of the immune response of a compound having the general formula I.

When the compounds of the invention are employed as immunomodulators, they can be formulated into oral dosage forms such as tablets, capsules and the like. The compounds can be administered alone or by combining them with conventional carriers, such as magnesium carbonate, magnesium stearate, talc, sugar, lactose, pectin, dextrin, starch, gelatin, tragacanth, methyl cellulose, sodium carboxymethyl cellulose, low melting wax, cocoa butter, and the like. Diluents, flavouring agents, solubilisers, lubricants, suspending agents, binders, tablet-disintegrating agents and the like may be employed. The compounds may be encapsulated with or without other carriers. In all cases the proportion of active ingredients in said compositions both solid and liquid will be at least sufficient to impart immunomodulatory activity thereto on oral administration. The compounds may also be injected parenterally, in which case they are used in the form of a sterile solution containing other solutes, for example, enough saline or glucose to make the solution isotonic.

The dosage requirements vary with the particular compositions employed, the route of administration, the severity of the symptoms presented and the particular subject being treated. Treatment will generally be initiated with small dosages less than the optimum dose of the compound. Thereafter the dosage is increased until the optimum effect under the circumstances is reached. In general, the compounds of the invention are most desirably administered at a concentration level that will generally afford effective results without causing any harmful or deleterious side effects. With large animals (about 70 kg. body weight), for injection administration the dose may be from about 25 milligrams to about 50 milligrams and for oral administration the dose may be from about 50 milligrams to about 200 milligrams and preferably from about 50 milligrams to about 100 milligrams per day either as a single unit dose, or if desired, the dosage may be divided into convenient subunits administered at convenient times throughout the day.

The immunomodulatory effect of the compounds of the invention may be demonstrated by standard pharmacological and histological procedures. The pharmacological procedures are described more fully in Examples 17 and 18 given hereinafter.

These procedures illustrate the ability of the compounds of the invention to exert an immunomodulatory effect, especially selectively on the cell-mediated immunity by directly measuring the effect of the compounds on the humoral and cell-mediated immunity, and include evaluation of the compounds in the Plescia anti-SRBC antibody production assay and evaluation in the mitogen activated murine T lymphocyte proliferation test.

Examples 1 to 5 and 7 to 16 illustrate the preparation of compounds having formula I and Example 6 illustrates the preparation of an intermediate compound having formula IV and being known from EP—A—21773.

Example 1

α-(2-Benzothiazolylthio)benzeneacetic acid

A. α-(2-Benzothiazolylthio)benzene acetic acid, hydrobromide

A solution of 16.7 g (0.10 m) 2-mercaptobenzothiazole and 21.5 g (0.10 m) α-bromo-α-phenylacetic acid in acetone is heated to reflux for 5 hours and the solvent is then removed. The residual oil is triturated in acetone and 31 g of solid collected. The compound melts at 192—4°C.

*Analysis:* $C_{15}H_{11}NO_2S_2 \cdot HBr$
Calculated: C, 47.12; H, 3.17; N, 3.66; Br, 20.90; S, 16.77
Found: C, 47.22; H, 3.06; N, 3.69; Br, 20.93; S, 16.73.

B. α-(2-Benzothiazolylthio)benzene acetic acid

The hydrobromide salt of A above is stirred in water at room temperature overnight, the solid collected and air dried. The crude material is recrystallised from acetonitrile to give the title compound in 70% yield and melting at 152—4°C.

*Analysis for:* $C_{15}H_{11}NO_2S_2$
Calculated: C, 59.78; H, 3.68; N, 4.65
Found: C, 59.81; H, 3.85; N, 4.77.

Example 2

α-(5-Chlorobenzothiazol-2-ylthio)benzene acetic acid

50.0 g (0.248 m) 5-chloro-2-mercaptobenzothiazole and 53.0 g (0.248 m) α-bromophenylacetic acid are dissolved in 1.5 l acetone and the solution is heated for 4 hours in the presence of 50 ml glacial acetic acid. The solution is concentrated to a smaller volume (about 200 ml) and the residual solid (90 g) is collected. The resulting salt is suspended in 1 l of water and the mixture is stirred at room temperature overnight. The collected solid is recrystallised from 2.5 l acetonitrile to give a total of 64 g (85% yield) of title compound melting at 190—2°C.

*Analysis for:* $C_{15}H_{10}ClNO_2S_2$
Calculated: C, 53.64; H, 3.00; N, 4.17
Found: C, 53.83; H, 3.13; N, 4.13.

Example 3

Sodium salt of α-(5-chlorobenzothiazol-2-ylthio)benzene acetic acid

To a solution of 10 g (0.03 m) of the compound of Example 2 in 600 ml of acetone is added an alcoholic NaOH (1.2 g (0.03 m) in 20 ml ethanol) solution. The precipitate is collected and recrystallised from water. 8.0 g (71% yield) of title compound is isolated as a monohydrate melting at 238—42°C (dec.).

*Analysis for:* $C_{15}H_9ClNO_2S_2Na\ H_2O$
Calculated: C, 47.93; H, 2.95; N, 3.73
Found: C, 47.7; H, 3.12; N, 3.79.

Example 4

α-(5-chlorobenzothiazol-2-ylthio)-α-(*p*-chlorophenyl)acetic acid

*Method A.* An acetone solution of 12.1 g (0.06 m) 5-chloro-2-mercaptobenzothiazole and 15.0 g (0.06 m) α-bromo-α-(p-chlorophenyl)-acetic acid is heated in the presence of 30 ml glacial acetic acid for 5 hours. After solvent removal, the residual solid is collected. This solid (9 g of the hydrobromic salt of the title compound) is stirred in water overnight and the solid is collected and air dried. The crude material is recrystallised from acetonitrile to give 4.7 g (21% yield) of the title compound melting at 157—8°C.

*Method B.* An acetone solution of the potassium salt of 5-chloro-2-mercaptobenzothiazole (0.05 m) and α-bromo-α-(*p*-chlorophenyl)-acetic acid (0.05 m) are heated to reflux for 3 days. The solid (KBr), formed during the reaction, is filtered off and the filtrate is concentrated, and the residual solid triturated with a mixture of acetone and hexane. The crude material is recrystallised from benzene to give 7.7 g (42% yield) of title compound melting at 157—8°C.

*Method C.* A methylene chloride solution of 10.0 g (0.05 m) 5-chloro-2-mercaptobenzothiazole, 12.5 g (0.05 m) α-bromo-α-(*p*-chlorophenyl)-acetic acid and 10.0 g (0.10 m) triethylamine is heated to reflux overnight. The reaction mixture is washed first with a dilute hydrochloric acid solution, then with water and finally dried over $MgSO_4$. After the methylene chloride is removed, the residual solid is triturated with acetonitrile to give 13.6 g (74% yield) of title compound melting at 157—8°C.

*Analysis for:* $C_{15}H_9Cl_2NO_2S_2$
Calculated: C, 48.65; H, 2.45; N, 3.78
Found: C, 48.69; H, 2.58; N, 3.79.

Example 5

α-(p-Chlorophenyl)-α-(benzothiazol-2-yl-thio) acetic acid

Following the procedure of Example 4, Method C and substituting 2-mercaptobenzothiazole for 5-chloro-2-mercaptobenzothiazole, the title compound is prepared in 34% yield and having a melting point of 185—7°C (dec.)

*Analysis for:* $C_{15}H_{10}ClNO_2S_2$
Calculated: C, 53.64; H, 3.00; N, 4.17
Found: C, 54.07; H, 3.17; N, 4.13.

Example 6

Preparation of 6-Chloro-2-phenylthiazolo[2,3-b]benzothiazol-3(2*H*)-one mesionic didehydro derivative

31 g (0.092 m) of the compound of Example 2 is suspended in 3.5 l methylene chloride and the mixture is heated to gentle reflux in the presence of 25 ml acetic anhydride. The solid is gradually dissolved, the solution turning reddish. After heating overnight, the solution is filtered and concentrated to 200 ml. The residual orange solid, weighing 29.5 g (quantitative yield), is collected and has a melting point of 215—6°C.

Example 7

α-(5-Chlorobenzothiazol-2-ylthio)benzene acetic acid

7 g of the compound of Example 6 is dissolved in 100 ml N,N-dimethylacetamide and the solution is allowed to stand at room temperature for 2 days in the presence of 5 ml water. The solution is diluted with 120 ml water and the resulting solid is collected and air dried. The crude material is recrystallised from 500 ml benzene. 5.9 g (80% yield) of purified title compound is obtained, having a melting point of 190—1°C.

*Analysis for:* $C_{15}H_{10}ClNO_2S_2$
Calculated: C, 53.64; H, 3.00; N, 4.17
Found: C, 53.83; H, 3.13; N, 4.13.

Example 8

α-(5-Chloro-2-benzothiazolylthio)benzene acetic acid, ethyl ester

8.0 g of the compound of Example 6 is dissolved in ethanol and the solution is warmed over a steam bath until the orange colour is discharged. The solvent is removed and oily residue is recrystallised from hexane. 6.6 g (73% yield) of title compound is obtained, having a melting point of 62—4°C.

*Analysis for:* $C_{17}H_{14}ClNO_2S_2$
Calculated: C, 56.11; H, 3.88; N, 3.85
Found: C, 56.05; H, 3.92; N, 3.84.

Example 9

α-(5-Chlorobenzothiazol-2-ylthio)-α-phenylacetamide

To 6.0 g (0.189 m) of the compound of Example 6 in 300 ml methylene chloride is introduced gaseous ammonia. The orange solid dissolves with discharge of the orange coloration by the precipitation of a white solid. After 15 minutes, the introduction of ammonia is stopped and the mixture is stirred for 30 minutes. The solid is collected and a further quantity of solid is recovered upon removal of the solvent from the filtrate. The combined solids are recrystallised from acetonitrile to give 5.0 g (79% yield) of title compound having a melting point of 181—3°C.

*Analysis for:* $C_{15}H_{11}ClN_2OS_2$
Calculated: C, 53.80; H, 3.31; N, 8.37
Found: C, 53.67; H, 3.41; N, 8.44.

Example 10

α-[(5-Chloro-2-benzothiazolyl)thio]-N-(2-hydroxyethyl)benzene acetamide

6.2 g (0.02 m) of the compound of Example 6 and 1.2 g (0.02 m) ethanolamine in a methylene chloride solution are warmed over a steam bath until dissolution is complete and the colouration discharged. The solution is filtered and the solvent removed. The residue is triturated with ether, the solid is collected and dried in a drying oven. 6.2 g (82% yield) of title compound, having a melting point of 148—9°C, is obtained.

*Analysis for:* $C_{17}H_{15}ClN_2O_2S_2$
Calculated: C, 53.88; H, 3.99; N, 7.40
Found: C, 54.03; H, 4.14; N, 7.46.

Example 11

α-[(5-Chloro-2-benzothiazolyl)thio]benzene acetic acid, [2-(acetylamino)ethyl]ester

3.18 g (0.01 m) of the compound of Example 6 and 1.03 g (0.01 m) N-acetylethanolamine are dissolved in methylene chloride and the solution is heated to reflux for 3 days. After removal of the solvent, the oily residue is recrystallised from 100 ml ether. 3.5 g (83% yield) of title compound, having a melting point of 107—8°C, is obtained.

*Analysis for:* $C_{19}H_{17}ClN_2O_3S_2$
Calculated: C, 54.21; H, 4.07; N, 6.66
Found: C, 53.95; H, 4.14; N, 6.59.

Example 12

α-[(5-Chloro-2-benzothiazolyl)thio]benzeneethanethioic acid, S-[2-(2-naphthalenylamino)-2-oxoethyl]ester

3.18 g (0.01 m) of the compound of Example 6 and 2.17 g (0.01 m) α-mercapto-N-(2-naphthyl)acetamide are dissolved in methylene chloride and the mixture is heated to reflux for 7 days. The progress of the reaction is monitored by TLC. After removal of solvent, the residue is recrystallised from ether. 2.3 g (42% yield) of title compound, having a melting point of 144—5°C, is obtained.

*Analysis for:* $C_{27}H_{19}ClN_2O_2S_3$
Calculated: C, 60.60; H, 3.58; N, 5.24
Found: C, 60.89; H, 3.78; N, 5.27.

Example 13

α-[(5-Chlorothiazolo[5,4-*b*]pyridin-2-yl)thio]-α-phenyl acetic acid

A methylene chloride solution of 4.05 g (0.02 m) 5-chloro-2-mercaptothiazolo[5,4-*b*]pyridine, 4.30 g (0.02 m) α-bromophenylacetic acid and 4.0 g (0.04 m) triethylamine is heated to gentle reflux overnight. The methylene chloride solution is extracted twice with a dilute hydrochloric acid solution and once with water and then dried over anhydrous $MgSO_4$. After the solvent is removed, the residual solid is recrystallised from acetonitrile. 5.0 g (74% yield) of title compound, melting at 175—7°C, is obtained.

*Analysis for:* $C_{14}H_9ClN_2O_2S_2$
Calculated: C, 49.92; H, 2.69; N, 8.32
Found: C, 49.97; H, 2.85; N, 8.37.

Example 14

α-[(5-Chlorothiazolo[5,4-*b*]pyridin-2-yl)thio]-α-(p-chlorophenyl) acetic acid

A methylene chloride solution of 6.0 g (0.03 m) of 5-chloro-2-mercaptothiazolo[5,4-*b*]pyridine, 7.5 g (0.03 m) α-bromo-α-(*p*-chlorophenyl) acetic acid and 6.0 g (0.06 m) triethylamine is heated to gentle reflux overnight. The solution is extracted twice with a dilute hydrochloric acid solution and then once with water. After drying over anhydrous $MgSO_4$, the solvent is removed and the residual solid is recrystallised from acetonitrile. 7.5 g (68% yield) of title compound, melting at 144—6°C, is obtained.

*Analysis for:* $C_{14}H_8Cl_2N_2O_2S_2$
Calculated: C, 45.29; H, 2.17; N, 7.55
Found: C, 44.87; H, 2.27; N, 7.59.

Example 15

α-Phenyl-α-[(4,5,6,7-tetrahydrobenzothiazol-2-yl)thio] acetic acid

A methylene chloride solution of 5.13 g (0.03 m) 2-mercapto-4,5,6,7-tetrahydrobenzothiazole, α-bromophenylacetic acid and 6.0 g (0.06 m) triethylamine is heated to gentle reflux overnight. The solution is extracted twice with a dilute hydrochloric acid solution, once with water and is then dried over anhydrous $MgSO_4$. The residual solid after solvent removal is recrystallised from acetonitrile. 7.0 g (77% yield) of title compound, melting at 118—20°C, is obtained.

*Analysis for:* $C_{15}H_{15}NO_2S_2$
Calculated: C, 58.99; N, 4.95; N, 4.59
Found: C, 59.0; H, 4.94; N, 4.52.

Example 16

α-[(6-Nitro-2-benzothiazolyl)thio]benzeneacetic acid

A methylene chloride solution of 5.0 g (0.0235 m) 2-mercapto-6-nitrobenzothiazole, 5.2 g (0.235 m) α-bromophenylacetic acid and 5.0 g (0.05 m) triethylamine is heated to gentle reflux overnight. The solution is washed twice with a dilute hydrochloric acid solution and once with water. The solution is dried over anhydrous $MgSO_4$ and then concentrated. The residue is recrystallised once from benzene and then from acetonitrile. 1.5 g (18% yield) of title compound, having a melting point of 152—5°C, is obtained.

*Analysis for:* $C_{15}H_{10}N_2O_4S_2$
Calculated: C, 52.01; H, 2.91; N, 8.09
Found: C, 51.72; H, 2.97; N, 8.01.

Example 17

The Plescia anti-SRBC (sheep red blood cells) antibody induction assay is a very sensitive test which is used to measure the ability of an immunomodulatory agent to augment or depress SRBC-induced production of SRBC-specific IgM and IgG antibodies. That is, this assay provides specific information regarding the ability of an immunomodulatory agent to induce the entire immune system to provide an enhanced or depressed antibody response to challenge with an antigen, which in the assay in SRBC. The effect of potential immunomodulatory compounds on T cells and their various subpopulations, as well as on B cells, can be studied in this assay.

The total assay procedure is essentially in three parts. In the first part, antigen-primed splenocytes (spleen cells) are prepared. C57B1/6J male mice are injected intraperitoneally with $10^7$SRBC (in pyrogen-free isotonic saline). Ten days later, the animals are sacrificed, their spleens excised and a suspensions of spleen cells is prepared therefrom. The suspension is prepared using 1 ml of tissue culture medium (Click medium supplemented with 10% fetal calf serum) per spleen and all suspensions are pooled into a single suspension. The number of viable nucleated spleen cells in the suspension is counted and adjusted to $10^7$/ml.

In the second part, 1 ml of the above suspension of prime spleen cells is added to sterile culture tubes to which is added 1 ml of a standard suspension of SRBC ($10^7$/ml of tissue culture medium). Stock solutions of compounds to be tested are prepared in tissue culture medium and from this appropriate dilutions are made to cover a range of concentrations. To each culture tube is added 0.1 ml of each compound dilution, while 0.1 ml of medium alone is added to the control tubes. The tubes are capped, mixed and then placed in a humidified −5% $CO_2$ incubator at 37°C for 6 days. The tubes are then centrifuged at 1000×g for about 10 minutes to sediment the cells and the supernatants decanted into test tubes.

In the third part, 2-fold serial dilutions of the supernatants, from 1/1 to 1/32, are made and 0.1 ml of each dilution is added to individual test tubes. To each tube there is added 0.1 ml of SRBC ($10^9$/ml), the tubes are mixed and allowed to stand at room temperature for 15—30 minutes. Then 0.1 ml complement (guinea pig serum diluted to contain a slight excess of complement) is added to each tube, mixed and the tubes are placed in a 37°C water bath for lysis of SRBC to occur. Only SRBC that have been sensitised by anti-SRBC antibody in the supernatant will lyse. 2.0 ml of isotonic saline is added to each tube, mixed and centrifuged to sediment unlysed cells and to recover the supernatant containing hemoglobin from the lysed SRBC. The supernatants' absorbance is measured at 541 nm.

Since the per cent of SRBC that is lysed is proportional to the amount of anti-SRBC antibody present in the test cell culture supernatant, and since lysis is measured in terms of the hemoglobin that is released from the lysed SRBC, it is the absorbance of the released hemoglobin that is measured in order to determine the quantity of anti-SRBC antibody present in the supernatant. Because the assay is most precise at the range of 50% lysis of SRBC in the presence of excess complement, the volume of supernatant that produces 50% lysis of SRBC, by definition, contains 1 unit of antibody called a 50% hemolytic unit (H50). One divided by this volume gives the number of H50's per ml of test supernatant. A standard curve of absorbance vs. H50/ml for control supernatant is then prepared, and the concentration of antibody in other test culture supernatants, for each dilution, is determined. The content of anti-SRBC antibody for the various test compounds at their various concentrations show whether the compound have caused stimulation or suppression of the immune response.

The results of this assay for several compounds of the invention are tabulated in Table 1, where the data is presented as H50 units of antibody/ml and % change from control.

TABLE 1

Experiment 1

| Compound (of Example No) | Quantity µg/ml | Units of Antibody | % Change from Control |
|---|---|---|---|
| Example 2 | 25 | 30.32 | −428 |
| | 2.5 | 84.96 | −147 |
| Example 4 | 25 | 62.3 | −200 |
| | 2.5 | 124.6 | 0 |
| Control | — | 124.9 | — |

Experiment 2

| Compound (of Example No) | Quantity µg/ml | Units of Antibody | % Change from Control |
|---|---|---|---|
| Example 4 | 25 | 47.53 | −348 |
| Example 3 | 25 | 48.26 | −343 |
| Control | — | 165.4 | — |

The results show that the compounds of the invention have a profound and significant depressive effect on the immune response, an effect which is further substantiated by the results in the mitogen activated murine T lymphocyte proliferation test.

Example 18

The mitogen activated murine T lymphocyte proliferation test is used to determine the effects of immunomodulatory agents on the proliferation of murine T cell populations. The test is run to determine whether test compounds will enhance or depress the proliferation of T cells, whose proliferation has been initially stimulated by a mitogen, such as Concanavallin A (Con A). To exclude the possibility that the test compounds act at Con A binding sites, the T cell proliferation can be studied in a system in which the test compounds are incubated with the T cells prior to the addition Con A instead of in a system in which test compounds and Con A are added to the cell cultures concomitantly.

The test procedure is as follows:

T lymphocytes are isolated from spleens of male CBA/J or NZB mice. Cell homogenates are prepared in Hank's balanced salt solution (HBSS). After removal of larger particles and repeated washing of the cells in HBSS they are suspended in minimum essential medium (MEM) and passed through a glass wool column to remove macrophages. The cells are then incubated on a nylon wool column at 37°C, 95% air, 5% $CO_2$, for 45 minutes. The non-adherant T lymphocytes are then eluted from the column, counted, and adjusted to $20 \times 10^6$ cells/ml. 50 µl of cells are cultured (37°C, 95% air, 5% air $CO_2$) with compound, or compound and mitogen (0.025 µg/culture of Concanavalin A) for 48 hours before the addition of 0.5 µCi of $^3$H-thymidine for the last 16 hours of culture. The total volume of the culture system is 200 µl. The cells are then harvested on a multiple automatic sample harvester (Mash II), the glass fibre discs placed in 10 ml of xylene base scintillation fluid, and counted for 1 minute in a liquid scintillation encounter.

The incubation experiments are conducted in two ways:

1) lymphocytes are incubated for 2 hours with test compounds, washed and cultured with different concentrations of Con A; and

2) lymphocytes are incubated for 24 hours with varied amounts of Con A, washed and then cultured with test compounds. The concentration of Con A used is either 0.04 or 0.05 µg/culture. The effects of some compounds of the invention are summarised as follows:

A). Sodium salt of α-(5-chlorobenzothiazol-2-ylthio)benzene acetic acid. When the compound is cultured concomitantly with Con A present in the amounts of 0.02; 0.04; 0.06; and 0.08 µg/culture, the compound inhibits the Con A induced blastogenesis at concentrations of 0.1, 1.0, 5 and 10 µg/culture. When the compound is cultured with lymphocytes prior to addition of 0.04 or 0.05 µg/culture of Con A, the compound failed to show inhibitory activity. When the compound is cultured with lymphocytes 24 hours after the cells are incubated with 0.04 or 0.05 µg/culture of Con A, at concentrations of >2 or 3 µg/culture the compound showed strong inhibition of lymphocyte blastogenesis.

B). α-(5-Chlorobenzothiazol-2-ylthio)-α-(*p*-chlorophenyl) acetic acid. When this compound is cultured concomitantly with Con A present in the amounts of 0.02; 0.03; 0.04 and 0/06 µg/culture, the compound

inhibits the Con A induced blastogenesis at concentrations >2 µg/culture. When the compound is cultured with lymphocytes prior to addition of 0.04 or 0.05 µg/culture of Con A, the compound shows consistent inhibitory activity at 3 and 5 µg/culture. When the compound is cultured with lymphocytes 24 hours after the cells are incubated with 0.04 or 0.05 µg/culture of Con A, at concentrations of >2 or 3 µg/culture the compound showed strong inhibition of blastogenesis.

These results clearly demonstrate that the compounds of the invention are strong and consistent inhibitors of T cell proliferation, which has been mitogenically induced. It is also clear that the compounds are able to perturb T lymphocyte blastogenesis when Con A binding sites are occupied, and so the compounds are not in competition with Con A for cell binding sites.

**Claims for the Contracting States: BE CH DE FR IT LI NL SE**

1. Use of a compound having the formula

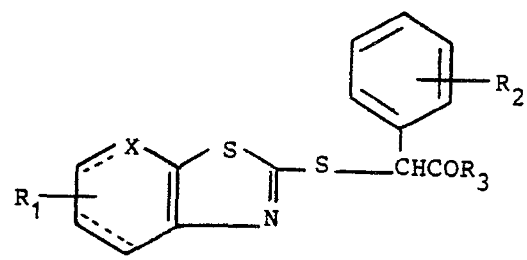

(I)

(wherein $R_1$ is hydrogen, halo, nitro, amino, $C_1$—$C_6$ alkyl, $C_1$—$C_6$ alkoxy, trifluoromethyl or hydroxy; $R_2$ is hydrogen, fluorine, chlorine, bromine, $C_1$—$C_6$ alkyl, $C_1$—$C_6$ alkoxy, amino, nitro or trifluoromethyl; $R_3$ is hydroxy, $C_1$—$C_6$ alkoxy, amino, hydroxy ($C_1$—$C_6$) alkylamino, N-($C_2$—$C_7$ alkanoyl)amino($C_1$—$C_6$)alkoxy) or N-arylcarbamoyl ($C_1$—$C_6$)alkylthio; X is CH or N, and the dotted lines represent optional double bonds in the indicated positions) or a pharmacologically acceptable salts thereof to manufacture a medicament for use as an immunomodulatory agent.

2. Use as claimed in Claim 1, wherein the ring containing X is iso-phenylene or o-phenylene substituted by halogen, hydroxy or $C_1$—$C_6$ alkoxy, $R_2$ is hydrogen or $C_1$—$C_6$ alkyl and $R_3$ is hydroxy, $C_1$—$C_6$ alkoxy or —$NHCH_2CH_2OH$.

3. Use as claimed in Claim 1, wherein the compound having formula I is selected from α-(2-benzothiazolylthio)benzene acetic acid; α-(5-chlorobenzothiazol-2-ylthio)benzeneacetic acid; α-(5-chloro-2-benzothiazolylthio)benzeneacetic acid, ethyl ester; and α-[(5-chloro-2-benzothiazolyl)thio]-N-(2-hydroxyethyl)benzeneacetamide.

4. A process for the preparation of a compound having the formula

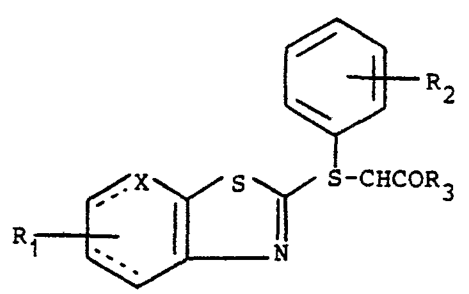

(I)

(wherein $R_1$, $R_2$, $R_3$, X and the dotted lines are as defined in Claim 1) or a pharmacologically acceptable salt thereof which comprises reacting a compound having the formula IV

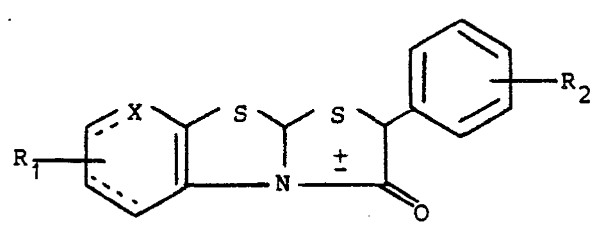

(IV)

with a compound having the formula $R_3H$ (wherein $R_1$, $R_2$, $R_3$, X and the dotted lines are as defined in Claim 1).

5. A process as claimed in Claim 4, wherein if $R_3$ is hydroxy then $R_1$ is nitro.

6. A compound having the formula

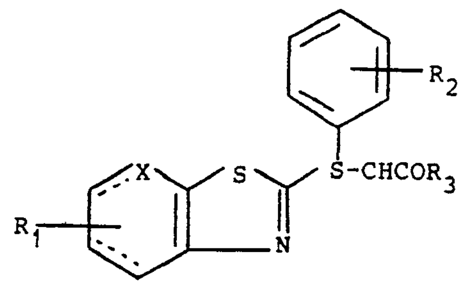

(I)

(wherein $R_1$, $R_2$, $R_3$, X and the dotted lines are as defined in Claim 1 subject to the provisos that (a) if $R_3$ is hydroxy then $R_1$ is nitro and (b) if $R_3$ is $C_1$—$C_6$ alkoxy or —$NHCH_2CH_2OH$ whilst $R_2$ is hydrogen or $C_1$—$C_6$ alkyl then the ring containing X is not o-phenylene or o-phenylene substituted by halogen, hydroxy or $C_1$—$C_6$ alkoxy) or a pharmacologically acceptable salt thereof.

7. A compound as claimed in Claim 6, wherein the compound having formula I is selected from α-(5-chlorobenzothiazol-2-ylthio)-α-phenylacetamide; α-[(5-chloro-2-benzothiazolyl)thio]benzene acetic acid, [2-(acetylamino)ethyl]ester; α-[(5-chloro-2-benzothiazolyl)thio]benzeneethanethioic acid, S-[2-(2-naphthalenylamino)-2-oxoethyl]ester; and α-[(6-nitro-2-benzothiazolyl)thio]benzene acetic acid.

8. A pharmaceutical composition which comprises a compound having the formula I as shown in Claim 1 (wherein $R_1$, $R_2$, $R_3$, X and the dotted lines are as defined in Claim 1 subject to the proviso that where the ring containing X is o-phenylene or o-phenylene substituted by halogen, hydroxy or $C_1$—$C_6$ alkoxy whilst $R_2$ is hydrogen or $C_1$—$C_6$ alkyl, then $R_3$ is not hydroxy, $C_1$—$C_6$ alkoxy or —$NHCH_2CH_2OH$) or a pharmacologically acceptable salt thereof in association or combination with a pharmaceutically acceptable carrier.

9. A composition as claimed in Claim 8, wherein the compound having formula I is selected from α-(5-chlorobenzothiazol-2-ylthio)-α-(*p*-chlorophenyl)acetic acid; α-(*p*-chlorophenyl)-α-(benzothiazol-2-ylthio)-acetic acid; α-(5-chlorobenzothiazol-2-ylthio)-α-phenylacetamide; α-[(5-chloro-2-benzothiazolyl)thio]-benzene acetic acid, [2-(acetylamino)ethyl]ester; α-[(5-chloro-2-benzothiazolyl)thio]benzeneethanethioic acid, S-[2-(2-naphthalenylamino)-2-oxoethyl]ester; α-[(5-chlorothiazolo[5,4-*b*]pyridin-2-yl)thio]-α-phenyl-acetic acid; α-[5-chlorothiazolo[5,4-*b*]pyridin-2-yl)thio]-α-(*p*-chlorophenyl)acetic acid; α-phenyl-α-[(4,5,6,7-tetrahydrobenzothiazol-2-yl)thio]acetic acid; and α-[(6-nitro-2-benzothiazolyl)thio]benzene acetic acid.

10. For use as a pharmaceutical, a compound having formula I as illustrated in Claim 1 (wherein $R_1$, $R_2$, $R_3$, X and the dotted lines are as defined in Claim 1, subject to the provisos that (a) $R_3$ is hydroxy, (b) $R_1$ is not nitro and (c) if the ring containing X is o-phenylene or o-phenylene substituted by halogen, hydroxy of $C_1$—$C_6$ alkoxy then $R_2$ is not hydrogen or $C_1$—$C_6$ alkyl) or a pharmacologically acceptable salt thereof.

11. A compound as claimed in Claim 10, wherein the compound having formula I is selected from α-(5-chlorobenzothiazol-2-ylthio)-α-(*p*-chlorophenyl)acetic acid; α-(*p*-chlorophenyl)-α-(benzothiazol-2-ylthio)-acetic acid; α-[(5-chlorothiazolo[5,4-*b*]pyridin-2-yl)thio]-α-phenylacetic acid; α-[5-chlorothiazolo[5,4-*b*]-pyridin-2-yl)thio]-α-(*p*-chlorophenyl)acetic acid; and α-phenyl-α-[(4,5,6,7-tetrahydrobenzothiazol-2-yl)thio]-acetic acid.

**Claims for the Contracting State: AT**

1. Use of a compound having the formula

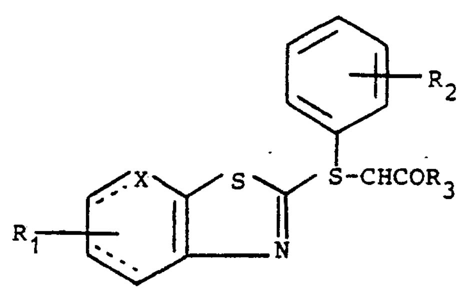

(I)

(wherein $R_1$ is hydrogen, halo, nitro, amino, $C_1$—$C_6$ alkyl, $C_1$—$C_6$ alkoxy, trifluoromethyl or hydroxy; $R_2$ is hydrogen, fluorine, chlorine, bromine, $C_1$—$C_6$ alkyl, $C_1$—$C_6$ alkoxy, amino, nitro or trifluoromethyl; $R_3$ is hydroxy, $C_1$—$C_6$ alkoxy, amino, hydroxy ($C_1$—$C_6$) alkylamino, N-($C_2$—$C_7$ alkanoyl)amino($C_1$—$C_6$)alkoxy) or N-arylcarbamoyl($C_1$—$C_6$)alkylthio; X is CH or N, and the dotted lines represent optional double bonds in the indicated positions) or a pharmacologically acceptable salt thereof to manufacture a medicament for use as an immunomodulatory agent.

2. Use as claimed in Claim 1, wherein the ring containing X is iso-phenylene or o-phenylene substituted by halogen, hydroxy or $C_1$—$C_6$ alkoxy, $R_2$ is hydrogen or $C_1$—$C_6$ alkyl and $R_3$ is hydroxy, $C_1$—$C_6$ alkoxy or —$NHCH_2CH_2OH$.

3. Use as claimed in Claim 1 or 2, wherein the medicament is prepared in the form of a tablet or capsule or in the form of a sterile solution containing other solutes.

4. Use as claimed in any one of Claims 1 to 3, wherein the medicament is prepared in unit dosage form.

5. Use as claimed in any one of Claims 1 to 4, wherein the compound having formula I is selected from α-(2-benzothiazolylthio)benzene acetic acid; α-(5-chlorobenzothiazol-2-ylthio)benzeneacetic acid; α-(5-chloro-2-benzothiazolylthio)benzeneacetic acid, ethyl ester; and α-[(5-chloro-2-benzothiazolyl)thio]-N-(2-hydroxyethyl)benzeneacetamide.

6. A process for the preparation of a compound having the formula

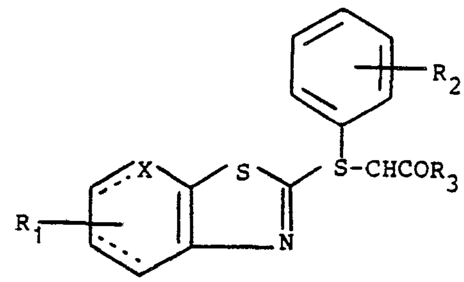

(I)

(wherein $R_1$, $R_2$, $R_3$, X and the dotted lines are as defined in Claim 1) or a pharmacologically acceptable salt thereof which comprises reacting a compound having the formula IV

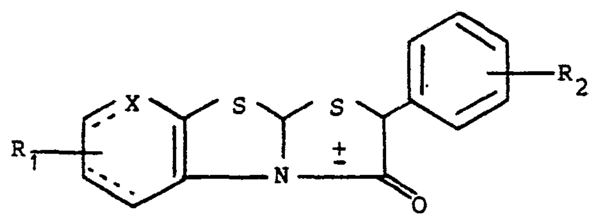

(IV)

with a compound having the formula $R_3H$ (wherein $R_1$, $R_2$, $R_3$, X and the dotted lines are as defined in Claim 1).

7. A process as claimed in Claim 6, wherein if $R_3$ is hydroxy then $R_1$ is nitro.

8. A process for the preparation of a compound having the formula

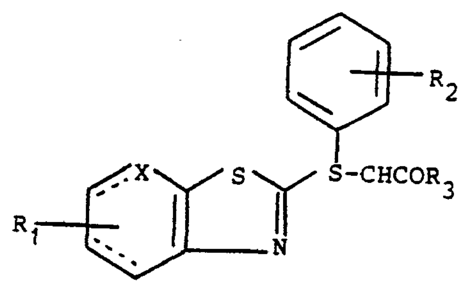

(I)

(wherein $R_1$, $R_2$, $R_3$, X and the dotted lines are as defined in Claim 1 subject to the provisos that (a) if $R_3$ is hydroxy then $R_1$ is nitro and (b) if $R_3$ is $C_1$—$C_6$ alkoxy or —$NHCH_2CH_2OH$ whilst $R_2$ is hydrogen or $C_1$—$C_6$ alkyl then the ring containing X is not o-phenylene or o-phenylene substituted by halogen, hydroxy or $C_1$—$C_6$ alkoxy) or a pharmacologically acceptable salt thereof, which comprises reacting a compound having the formula II

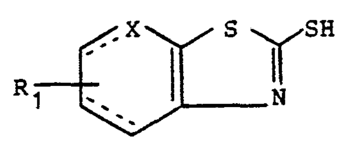

(II)

(wherein $R_1$, X and the dotted lines are as defined above) or a salt thereof with a compound having the formula

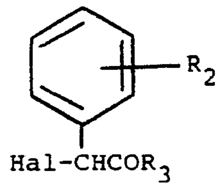

(III)

(wherein Hal is chlorine or bromine and $R_2$ and $R_3$ are as defined above) or a salt thereof.

9. A process for the preparation of a pharmaceutical composition which comprises bringing a compound having the formula I as shown in Claim 1 (wherein $R_1$, $R_2$, $R_3$ X and the dotted lines are as defined in claim 1 subject to the proviso that where the ring containing X is o-phenylene or o-phenylene substituted by halogen, hydroxy or $C_1$—$C_6$ alkoxy whilst $R_2$ is hydrogen or $C_1$—$C_6$ alkyl, then $R_3$ is not hydroxy, $C_1$—$C_6$ alkoxy or —$NHCH_2CH_2OH$) or a pharmacologically acceptable salt thereof into association or combination with a pharmaceutically acceptable carrier.

10. A process as claimed in Claim 9, wherein the compound having formula I is selected from α-(5-chlorobenzothiazol-2-ylthio)-α-(*p*-chlorophenyl)acetic acid; α-(*p*-chlorophenyl)-α-(benzothiazol-2-ylthio)-acetic acid; α-(5-chlorobenzothiazol-2-ylthio)-α-phenylacetamide; α-[(5-chloro-2-benzothiazolyl)thio]-benzene acetic acid, [2-(acetylamino)ethyl]ester; α-[(5-chloro-2-benzothiazolyl)thio]benzeneethanethioic acid, S-[2-(2-naphthalenylamino)-2-oxoethyl]ester; α-[(5-chlorothiazolo[5,4-*b*]pyridin-2-yl)thio]-α-phenyl-acetic acid; α-[5-chlorothiazolo[5,4-*b*]pyridin-2-yl)thio]-α-(*p*-chlorophenyl)acetic acid; α-phenyl-α-[(4,5,6,7-tetrahydrobenzothiazol-2-yl)thio]acetic acid; and α-[(6-nitro-2-benzothiazolyl)thio]benzene acetic acid.

11. A process for the preparation of a compound having formula I as illustrated in Claim 8 or a pharmacologically acceptable salt thereof for use as a pharmaceutical, which comprises reacting a compound having formula II as illustrated in Claim 8 with a compound having formula III as illustrated in claim 8, wherein $R_1$, $R_2$, $R_3$, X and the dotted lines are as defined in Claim 1, subject to the provisos that (a) $R_3$ is hydroxy, $R_1$ is not nitro and (b) if $R_2$ is hydrogen or $C_1$—$C_6$ alkyl then the ring containing X is not o-phenylene or o-phenylene substituted by halogen, hydroxy or $C_1$—$C_6$ alkoxy.

**Claims for the Contracting State: LU**

1. A pharmaceutical composition comprising a compound having the formula I

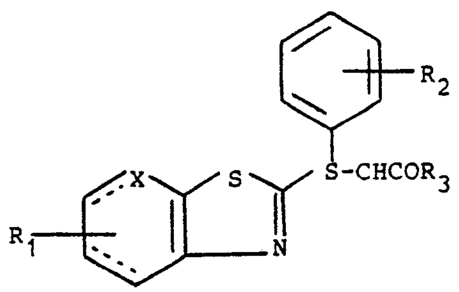

(I)

wherein $R_1$ is hydrogen, halo, nitro, amino, $C_1$—$C_6$ alkyl, $C_1$—$C_6$ alkoxy, trifluoromethyl or hydroxy; $R_2$ is hydrogen, fluorine, chlorine, bromine, $C_1$—$C_6$ alkyl, $C_1$—$C_6$ alkoxy, amino, nitro or trifluoromethyl; $R_3$ is hydroxy, $C_1$—$C_6$ alkoxy, amino, hydroxy($C_1$—$C_6$)alkylamino, N-($C_2$—$C_7$ alkanoyl)amino($C_1$—$C_6$ alkoxy) or N-arylcarbamoyl($C_1$—$C_6$)alkylthio; X is CH or N, and the dotted lines represent optional double bonds in the 5, 6 and 7, 8- indicated positions, or a pharmacologically acceptable salt thereof in association or combination with a pharmaceutically acceptable carrier.

2. A composition as claimed in Claim 1, in unit dosage form.

3. A composition as claimed in Claim 1 or 2 in the form of one or more tablets or capsules or in the form of a sterile solution containing other solutes.

4. A composition as claimed in any one of Claims 1 to 3, wherein the compound having formula I is selected from α-(2-benzothiazolylthio)benzene acetic acid; α-(5-chlorobenzothiazol-2-ylthio)benzeneacetic acid; α-(5-chlorobenzothiazol-2-ylthio)-α-(*p*-chlorophenyl)acetic acid; α-(*p*-chlorophenyl)-α-(benzothiazol-2-ylthio)acetic acid; α-(5-chloro-2-benzothiazolyl)benzene acetic acid, ethyl ester; α-(5-chlorobenzothiazol-2-ylthio)-α-phenylacetamide; α-[(5-chloro-2-benzothiazolyl)thio]-N-(2-hydroxyethyl)benzene-acetamide; α-[(5-chloro-2-benzothiazolyl)thio]benzene acetic acid, [2-(acetylamino)ethyl]ester; α-[(5-chloro-2-benzothiazolyl)thio]benzeneethanethioic acid, S-[2-(2-naphthalenylamino)-2-oxoethyl]ester; α-[(5-chlorothiazolo[5,4-*b*]pyridin-2-yl)thio]-α-phenylacetic acid; α-[5-chlorothiazolo[5,4-*b*]pyridin-2-yl)thio]-α-(*p*-chlorophenyl)acetic acid; α-phenyl-α-[(4,5,6,7-tetrahydrobenzothiazol-2-yl)thio]acetic acid and α-[(6-nitro-2-benzothiazolyl)thio]benzene acetic acid.

5. For use as an immunomodulating agent, a compound having the formula I

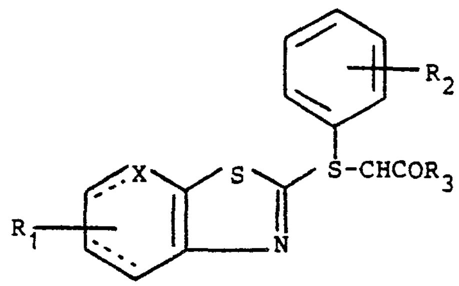

(I)

(wherein $R_1$, $R_2$, $R_3$, X and the dotted lines are as defined in Claim 1) or a pharmacologically acceptable salt thereof.

6. A compound as claimed in Claim 5, which is selected from α-(2-benzothiazolylthio)benzene acetic acid; α-(5-chlorobenzothiazol-2-ylthio)benzene acetic acid; α-(5-chlorobenzothiazol-2-ylthio)-α-(*p*-chlorophenyl)acetic acid; α-(*p*-chlorophenyl)-α-(benzothiazol-2-ylthio)acetic acid; α-[(5-chlorobenzothiazolo[5,4-*b*]pyridin-2-yl)thio]-α-phenylacetic acid; α-(5-chlorothiazolo[5,4-*b*]pyridin-2-yl)thio-α-(*p*-chlorophenyl)-acetic acid; α-phenyl-α-[(4,5,6,7-tetrahydrobenzothiazol-2-yl)thio]acetic acid and the pharmacologically acceptable salt thereof.

7. A compound having the formula I

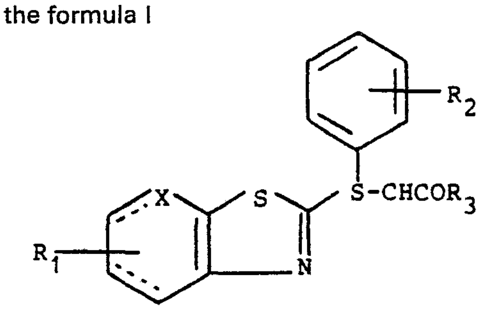

(I)

or a pharmacologically acceptable salt thereof, wherein $R_1$, $R_2$, $R_3$, X and the dotted lines are as defined in Claim 1 subject to the proviso that, where $R_3$ is hydroxy, then $R_1$ is nitro.

8. A compound as claimed in Claim 7, which is selected from α-[(6-nitro-2-benzothiazolyl)thio]-benzeneacetic acid and the pharmacologically acceptable salts thereof; α-[(5-chloro-2-benzothiazolyl)thio]benzene ethanethioic acid, S-[2-(2-naphthalenylamino)-2-oxoethyl]ester; α-[(5-chloro-2-benzothiazolyl)thio]benzeneacetic acid, [2-(acetylamino)ethyl]ester; α-[(5-chloro-2-benzothiazolyl)thio]-N-(2-hydroxyethyl)benzeneacetamide; and α-(5-chloro-2-benzothiazolylthio)benzeneacetic acid, ethyl ester.

9. A compound as claimed in Claim 7, which is α-(5-chlorobenzothiazol-2-ylthio)-α-phenylacetamide.

10. A process for the preparation of a compound having the formula:

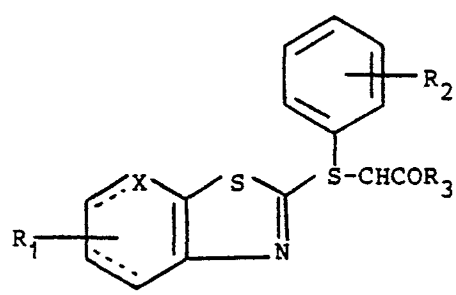

(I)

(wherein $R_1$, $R_2$, $R_3$, X and the dotted lines are as defined in Claim 1) or a pharmacologically acceptable salt thereof, which comprises

(a) reacting a compound having the formula II

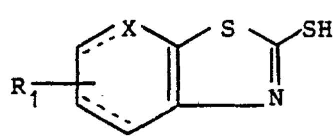

(II)

(wherein $R_1$, X and the dotted lines are as defined in Claim 1) or a salt thereof with a compound having the formula

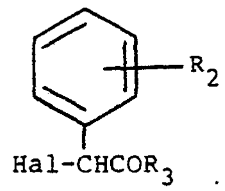

(III)

(wherein Hal is chlorine or bromine and $R_2$ and $R_3$ are as defined in Claim 1 subject to the proviso that, where $R_3$ is hydroxy, then $R_1$ in formula II is nitro) or a salt thereof; or

(b) reacting a compound having the formula IV

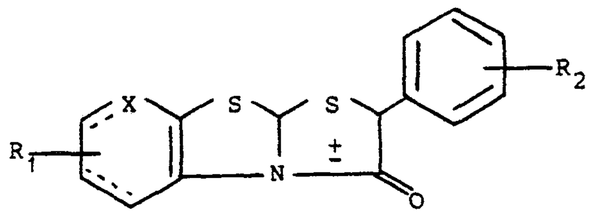

(IV)

with a compound having the formula $R_3H$ (wherein $R_1$, $R_2$, $R_3$, X and the dotted lines are as defined in Claim 1).

**Patentansprüche für die Vertragsstaaten: BE CH DE FR IT LI NL SE**

1. Verwendung einer Verbindung der Formel

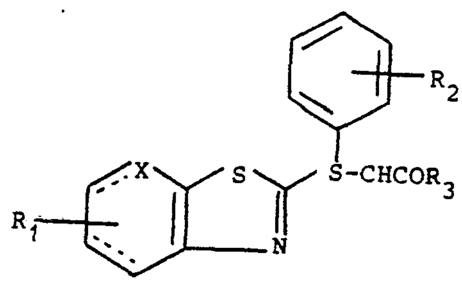

(I)

(worin $R_1$ Wasserstoff, Halogen, Nitro, Amino, $C_1$—$C_6$-Alkyl, $C_1$—$C_6$-Alkoxy, Trifluormethyl oder Hydroxy bedeutet; $R_2$ Wasserstoff, Fluor, Chlor, Brom, $C_1$—$C_6$-Alkyl, $C_1$—$C_6$-Alkoxy, Amino, Nitro oder Trifluormethyl darstellt; $R_3$ Hydroxy, $C_1$—$C_6$-Alkoxy, Amino, Hydroxy-($C_1$—$C_6$)-alkylamino, N-($C_2$—$C_7$-Alkanoyl)amino-($C_1$—$C_6$-alkoxy) oder N-Arylcarbamoyl-($C_1$—$C_6$)-alkylthio ist; X die Bedeutung CH oder N hat und die strichlierten Linien gegebenenfalls vorhandene Doppelbindungen in den angegebenen Stellungen sind) oder eines pharmakologisch annehmbaren Salzes hievon zur Herstellung eines Medikaments zur Verwendung als immunmodulierendes Mittel.

2. Verwendung, wie in Anspruch 1 beansprucht, wobei der X enthaltende Ring Isophenylen oder o-Phenylen substituiert durch Halogen, Hydroxy oder $C_1$—$C_6$-Alkoxy ist, $R_2$ Wasserstoff oder $C_1$—$C_6$-Alkyl bedeutet und $R_3$ Hydroxy, $C_1$—$C_6$-Alkoxy oder —$NHCH_2CH_2OH$ darstellt.

3. Verwendung, wie in Anspruch 1 beansprucht, wobei die Verbindung der Formel (I) ausgewählt ist aus α-(2-Benzothiazolylthio)-benzolessigsäure; α-(5-Chlorbenzothiazol-2-ylthio)-benzolessigsäure; α-(5-Chlor-2-benothiazolylthio)-benzolessigsäure-äthylester und α-[(5-Chlor-2-benzothiazolyl)-thio]-N-(2-hydroxyäthyl)-benzolacetamid.

4. Verfahren zur Herstellung einer Verbindung der Formel

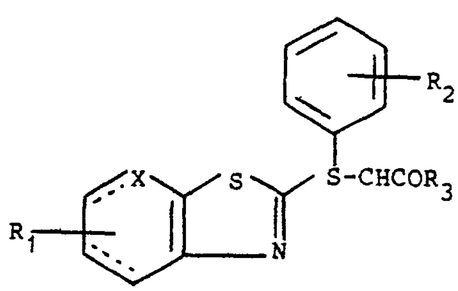

(I)

(worin $R_1$, $R_2$, $R_3$, X und die strichlierten Linien wie in Anspruch 1 definiert sind) oder eines pharmakologisch annehmbaren Salzes hievon, welches das Umsetzen einer Verbindung der Formel (IV)

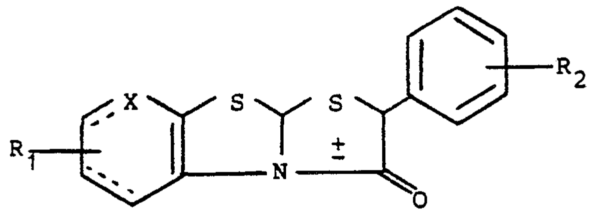

(IV)

mit einer Verbindung der Formel $R_3H$ (worin $R_1$, $R_2$, $R_3$, X und die strichlierten Linien wie in Anspruch 1 definiert sind) umfaßt.

5. Verfahren nach Anspruch 4, worin, wenn $R_3$ Hydroxy ist, $R_1$ Nitro bedeutet.

6. Eine Verbindung der Formel

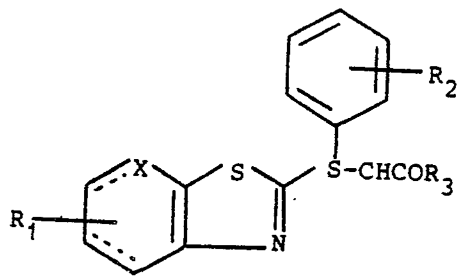

(I)

(worin $R_1$, $R_2$, $R_3$, X und die strichlierten Linien wie in Anspruch 1 definiert sind, mit den Maßgaben, daß (a) wenn $R_3$ Hydroxy ist, $R_1$ Nitro bedeutet, und (b) wenn $R_3$ $C_1$—$C_6$-Alkoxy oder —$NHCH_2CH_2OH$ ist, während $R_2$ Wasserstoff oder $C_1$—$C_6$-Alkyl darstellt, dann der X enthaltende Ring nicht o-Phenylen oder o-Phenylen substituiert durch Halogen, Hydroxy oder $C_1$—$C_6$-Alkoxy ist) oder ein pharmakologisch annehmbares Salz hievon.

7. Eine Verbindung, wie in Anspruch 6 beansprucht, wobei die Verbindung der Formel (I) ausgewählt ist aus α-(5-Chlorbenzothiazol-2-ylthio)-α-phenylacetamid; α-[(5-Chlor-2-benzothiazolyl)-thio]-benzolessigsäure-[2-(acetylamino)-äthyl]-ester; α-[(5-Chlor-2-benzothiazolyl)-thio]-benzoläthanthiosäure-S-[2-(2-naphthalenylamino)-2-oxoäthyl]-ester; und α-[(6-Nitro-2-benzothiazolyl)-thio] benzolessigsäure.

8. Pharmazeutische Zusammensetzung, die eine Verbindung der Formel (I), wie in Anspruch 1 gezeigt (worin $R_1$, $R_2$, $R_3$, X und die strichlierten Linien wie in Anspruch 1 definiert sind, mit der Maßgabe, daß, wenn der X enthaltende Ring o-Phenylen oder o-Phenylen substituiert durch Halogen, Hydroxy oder $C_1$—$C_6$-Alkoxy bedeutet, während $R_2$ Wasserstoff oder $C_1$—$C_6$-Alkyl ist, dann $R_3$ nicht Hydroxy, $C_1$—$C_6$-Alkoxy oder —$NHCH_2OH_2$ ist) oder ein pharmakologisch annehmbares Salz hievon in Vereinigung oder Kombination mit einem pharmazeutisch annehmbaren Träger umfaßt.

9. Zusammensetzung, wie in Anspruch 8 beansprucht, worin die Verbindung der Formel (I) ausgewählt ist aus α-(5-Chlorbenzothiazol-2-ylthio)-α-(p-chlorphenyl)-essigsäure; α-(p-Chlorphenyl)-α-(benzothiazol-2-ylthio)-essigsäure; α-(5-Chlorbenzothiazol-2-ylthio)-α-phenylacetamid; α-[(5-Chlor-2-benzothiazolyl)-thio]-benzolessigsäure-[2-(acetylamino)-äthyl]-ester; α-[(5-Chlor-2-benzothiazolyl)-thio]-benzoläthanthiosäure-S-[2-(2-naphthalenylamino)-2-oxoäthyl]-ester; α-[(5-Chlorthiazol[5,4-b]-pyridin-2-yl)-thio]-α-phenylessigsäure; α-[5-Chlorthiazol[5,4-b]-pyridin-2-yl)-thio]-α-(p-chlorphenyl)-essigsäure; α-Phenyl-α-[(4,5,6,7-tetrahydrobenzothiazol-2-yl)-thio]-essigsäure; und α-[(6-Nitro-2-benzothiazolyl)-thio]-benzolessigsäure.

10. Eine Verbindung der Formel (I), wie in Anspruch 1 illustriert (worin $R_1$, $R_2$, $R_3$, X und die strichlierten Linen wie in Anspruch 1 definiert sind, mit den Maßgaben, daß (a) $R_3$ Hydroxy ist, (b) $R_1$ nicht Nitro bedeutet und (c), wenn der X enthaltende Ring o-Phenylen oder o-Phenylen substituiert durch Halogen, Hydroxy oder $C_1$—$C_6$-Alkoxy ist, dann $R_2$ nicht Wasserstoff oder $C_1$—$C_6$-Alkyl bedeutet), oder ein pharmakologisch annehmbares Salz hievon zur Verwendung als Pharmazeutikum.

11. Eine Verbindung, wie in Anspruch 10 beansprucht, worin die Verbindung der Formel (I) ausgewählt ist aus α-(5-Chlorbenzothiazol-2-ylthio)-α-(p-chlorphenyl)-essigsäure; α-(p-Chlorphenyl)-α-(benzothiazol-2-ylthio)-essigsäure; α-[(5-Chlorthiazol[5,4-b]pyridin-2-yl)-thio]-α-phenylessigsäure; α-[5-Chlorthiazol[5,4-b]pyridin-2-yl)-thio]-α-(p-chlorphenyl)-essigsäure und α-Phenyl-α-[(4,5,6,7-tetrahydrobenzothiazol-2-yl)-thio]-essigsäure.

**Patentansprüche für den Vertragsstaat: AT**

1. Verwendung einer Verbindung der Formel

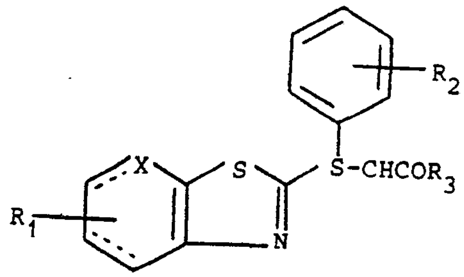

(I)

(worin $R_1$ Wasserstoff, Halogen, Nitro, Amino, $C_1$—$C_6$-Alkyl, $C_1$—$C_6$-Alkoxy, Trifluormethyl oder Hydroxy bedeutet; $R_2$ Wasserstoff, Fluor, Chlor, Brom, $C_1$—$C_6$-Alkyl, $C_1$—$C_6$-Alkoxy, Amino, Nitro oder Trifluormethyl darstellt; $R_3$ Hydroxy, $C_1$—$C_6$-Alkoxy, Amino, Hydroxy-($C_1$—$C_6$)-alkylamino, N-($C_2$—$C_7$-Alkanoyl)amino-($C_1$—$C_6$-alkoxy) oder N-Arylcarbamoyl-($C_1$—$C_6$)-alkylthio ist; X die Bedeutung CH oder N hat und die strichlierten Linien gegebenenfalls vorhandene Doppelbindungen in den angegebenen Stellungen sind) oder eines pharmakologisch annehmbaren Salzes hievon zur Herstellung eines Medikaments zur Verwendung als immunmodulierendes Mittel.

2. Verwendung, wie in Anspruch 1 beansprucht, wobei der X enthaltende Ring Isophenylen oder o-Phenylen substituiert durch Halogen, Hydroxy oder $C_1$—$C_6$-Alkoxy ist, $R_2$ Wasserstoff oder $C_1$—$C_6$-Alkyl bedeutet und $R_3$ Hydroxy, $C_1$—$C_6$-Alkoxy oder —$NHCH_2CH_2OH$ darstellt.

3. Verwendung, wie in Anspruch 1 oder 2 beansprucht, wobei das Medikament in Form einer Tablette oder Kapsel oder in Form einer sterilen Lösung, die andere gelöste Stoffe enthält, hergestellt wird.

4. Verwendung, wie in einem der Ansprüche 1 bis 3 beansprucht, wobei das Medikament in Einheitsdosierungsform hergestellt wird.

5. Verwendung, wie in einem der Ansprüche 1 bis 4 beansprucht, wobei die Verbindung der Formel (I) ausgewählt ist aus α-(2-Benzothiazolylthio)-benzolessigsäure; α-(5-Chlorbenzothiazol-2-ylthio)-benzolessigsäure; α-(5-Chlor-2-benzothiazolylthio)-benzolessigsäure-äthylester und α-[(5-Chlor-2-benzothiazolyl)-thio]-N-(2-hydroxyäthyl)-lbenzolacetamid.

6. Verfahren zur Herstellung einer Verbindung der Formel

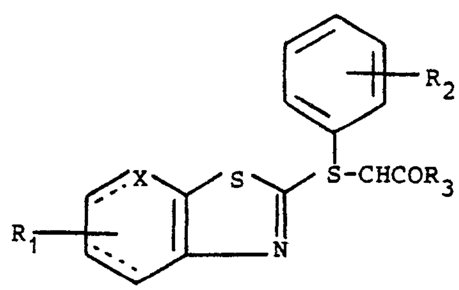

(I)

(worin $R_1$, $R_2$, $R_3$, X und die strichlierten Linien wie in Anspruch 1 definiert sind) oder eines pharmakologisch annehmbaren Salzes hievon, welches das Umsetzen einer Verbindung der Formel (IV)

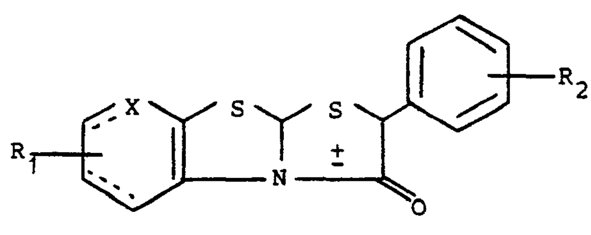

(IV)

mit einer Verbindung der Formel $R_3H$ (worin $R_1$, $R_2$, $R_3$, X und die strichlierten Linien wie in Anspruch 1 definiert sind) umfaßt.

7. Verfahren nach Anspruch 6, worin, wenn $R_3$ Hydroxy ist, $R_1$ Nitro bedeutet.

8. Verfahren zur Herstellung einer Verbindung der Formel

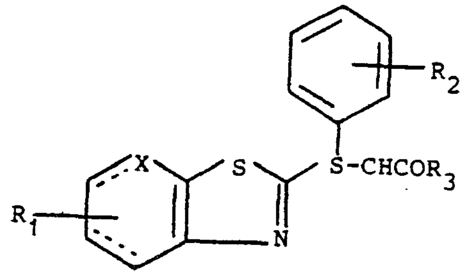

(I)

(worin $R_1$, $R_2$, $R_3$, X und die strichlierten Linien wie in Anspruch 1 definiert sind, mit den Maßgaben, daß (a) wenn $R_3$ Hydroxy ist, $R_1$ Nitro bedeutet, und (b) wenn $R_3$ $C_1$—$C_6$-Alkoxy oder —$NHCH_2CH_2OH$ darstellt, während $R_2$ Wasserstoff oder $C_1$—$C_6$-Alkyl ist, dann der X enthaltende Ring nicht o-Phenylen oder o-Phenylen substituiert durch Halogen, Hydroxy oder $C_1$—$C_6$-Alkoxy ist) oder eines pharmakologisch annehmbaren Salzes hievon, welches das Umsetzen einer Verbindung der Formel (II)

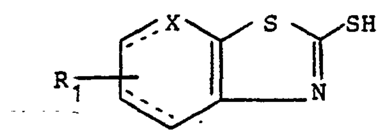

(II)

(worin $R_1$, X und die strichlierten Linien wie oben definiert sind) oder eines Salzes hievon mit einer Verbindung der Formel

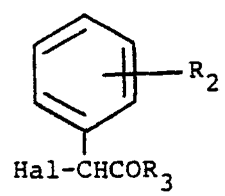

(III)

(worin Hal Chlor oder Brom bedeutet und $R_2$ und $R_3$ wie oben definiert sind) oder einem Salz hievon umfaßt.

9. Verfahren zur Herstellung einer pharmazeutischen Zusammensetzung, welches die Vereinigung oder Kombination einer Verbindung der Formel (I), wie in Anspruch 1 gezeigt (worin $R_1$, $R_2$, $R_3$, X und die strichlierten Linien wie in Anspruch 1 definiert sind, mit der Maßgabe, daß, wenn der X enthaltende Ring o-Phenylen oder o-Phenylen substituiert mit Halogen, Hydroxy oder $C_1$—$C_6$-Alkoxy ist, wobei $R_2$ Wasserstoff oder $C_1$—$C_6$-Alkyl bedeutet, dann $R_3$ nicht Hydroxy, $C_1$—$C_6$-Alkoxy oder —$NHCH_2CH_2OH_2$ ist) oder eines pharmakologisch annehmbaren Salzes hievon mit einem pharmazeutisch annehmbaren Träger umfaßt.

10. Verfahren, wie in Anspruch 9 beansprucht, worin die Verbindung der Formel (I) ausgewählt wird aus α-(5-Chlorbenzothiazol-2-ylthio)-α-(p-chlorphenyl)-essigsäure; α-(p-Chlorphenyl)-α-(benzothiazol-2-ylthio)-essigsäure; α-(5-Chlorbenzothiazol-2-ylthio)-α-phenylacetamid; α-[(5-Chlor-2-benzothiazolyl)-thio]-benzolessigsäure-[2-(acetylamino)-äthyl]-ester; α-[(5-Chlor-2-benzothiazolyl)-thio]-benzoläthanthiosäure-S-[2-(2-naphthalenylamino)-2-oxoäthyl]-ester; α-[(5-Chlorthiazol[5,4-b]-pyridin-2-yl)-thio]-α-phenylessigsäure; α-[5-Chlorthiazol[5,4-b]-pyridin-2-yl)-thio]-α-(p-chlorphenyl)-essigsäure; α-Phenyl-α-[(4,5,6,7-tetrahydrobenzothiazol-2-yl)-thio]-essigsäure; und α-[(6-Nitro-2-benzothiazolyl)-thio]-benzolessigsäure.

11. Verfahren zur Herstellung einer Verbindung der Formel (I), wie in Anspruch 8 illustriert, oder eines pharmakologisch annehmbaren Salzes hievon zur Verwendung als Pharmazeutikum, welches das Umsetzen einer Verbindung der Formel (II), wie in Anspruch 8 illustriert, mit einer Verbindung der Formel (III), wie in Anspruch 8 illustriert, worin $R_1$, $R_2$, $R_3$, X und die strichlierten Linien wie in Anspruch 1 definiert sind, mit den Maßgaben, daß (a) $R_3$ Hydroxy ist, $R_1$ nicht Nitro bedeutet, und (b) wenn $R_2$ Wasserstoff oder $C_1$—$C_6$-Alkyl ist, dann der X enthaltende Ring nicht o-Phenylen oder o-Phenylen substituiert durch Halogen, Hydroxy oder $C_1$—$C_6$-Alkoxy darstellt, umfaßt.

**Patentansprüche für den Vertragsstaaten: LU**

1. Pharmazeutische Zusammensetzung, die eine Verbindung der Formel

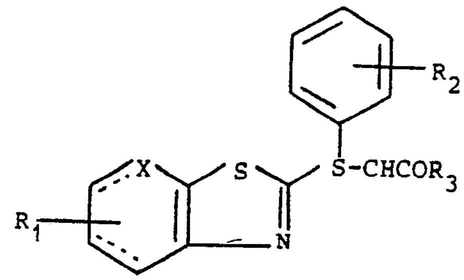

(I)

(worin $R_1$ Wasserstoff, Halogen, Nitro, Amino, $C_1$—$C_6$-Alkyl, $C_1$—$C_6$-Alkoxy, Trifluormethyl oder Hydroxy bedeutet; $R_2$ Wasserstoff, Fluor, Chlor, Brom, $C_1$—$C_6$-Alkyl, $C_1$—$C_6$-Alkoxy, Amino, Nitro oder Trifluormethyl darstellt; $R_3$ Hydroxy, $C_1$—$C_6$-Alkoxy, Amino, Hydroxy-($C_1$—$C_6$)-alkylamino, N-($C_2$—$C_7$-Alkanoyl)amino-($C_1$—$C_6$-alkoxy) oder N-Arylcarbamoyl-($C_1$—$C_6$)-alkylthio ist; X die Bedeutung CH oder N hat und die strichlierten Linien gegebenenfalls vorhandene Doppelbindungen in den angegebenen Stellungen 5,6 und 7,8 sind, oder ein pharmakologisch annehmbares Salz hievon in Vereinigung oder Kombination mit einem pharmazeutisch annehmbaren Träger umfaßt.

2. Zusammensetzung, wie in Anspruch 1 beansprucht, in Einheitsdosierungsform.

3. Zusammensetzung, wie in Anspruch 1 oder 2 beansprucht, in Form einer oder mehrerer Tabletten oder Kapseln oder in Form einer sterilen Lösung, die andere gelöste Stoffe enthält.

4. Zusammensetzung, wie in einem der Ansprüche 1 bis 3 beansprucht, worin die Verbindung der Formel (I) ausgewählt ist aus α-(2-Benzothiazolylthio)-benzolessigsäure; α-(5-Chlorbenzothiazol-2-ylthio)-benzolessigsäure; α-(5-Chlorbenzothiazol-2-ylthio)-α-(p-chlorphenyl)-essigsäure; α-(p-Chlorphenyl)-α-(benzothiazol-2-ylthio)-essigsäure; α-(5-Chlor-2-benzothiazolylthio)-benzolessigsäure-äthylester; α-(5-Chlorbenzothiazol-2-ylthio)-α-phenylacetamid; α-[(5-Chlor-2-benzothiazolyl)-thio]-N-(2-hydroxyäthyl)-benzolacetamid; α-[(5-Chlor-2-benzothiazolyl)-thio]-benzolessigsäure-[2-(acetylamino)-äthyl]-ester; α-[(5-Chlor-2-benzothiazolyl)-thio]-benzoläthanthiosäure-S-[2-(2-naphthalenylamino)-2-oxoäthyl]-ester; α-[(5-Chlorthiazol-[5,4-b]pyridin-2-yl)-thio]-α-phenylessigsäure; α-[5-Chlorthiazol[5,4-b]pyridin-2-yl)-thio]-α-(p-chlorphenyl)-essigsäure; α-Phenyl-α-[(4,5,6,7-tetrahydrobenzothiazol-2-yl)-thio]-essigsäure und α-[(6-Nitro-2-benzothiazolyl)-thio]-benzolessigsäure.

5. Eine Verbindung der Formel (I)

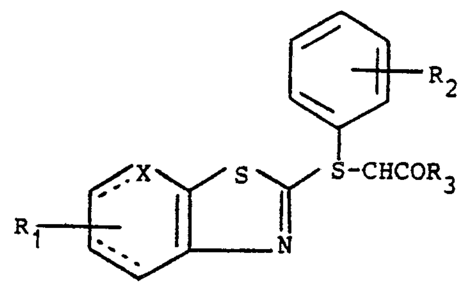

(I)

(worin $R_1$, $R_2$, $R_3$, X und die strichlierten Linien wie in Anspruch 1 definiert sind) oder ein pharmakologisch annehmbares Salz hievon zur Verwendung als immunmodulierendes Mittel.

6. Eine Verbindung, wie in Anspruch 5 beansprucht, die ausgewählt ist aus α-(2-Benzothiazolylthio)-benzolessigsäure; α-(5-Chlorbenzothiazol-2-ylthio)-benzolessigsäure; α-(5-Chlorbenzothiazol-2-ylthio)-α-(p-chlorphenyl)-essigsäure; α-(p-Chlorphenyl)-α-(benzothiazol-2-ylthio)-essigsäure; α-[(5-Chlorthiazol[5,4-b)pyridin-2-yl)-thio]-α-phenylessigsäure; α-(5-Chlorthiazol[5,4-b]pyridin-2-yl)-thio-α-(p-chlorphenylessigsäure; α-Phenyl-α-[(4,5,6,7-tetrahydrobenzothiazol-2-yl)-thio]-essigsäure und den pharmakologisch annehmbaren Salzen hievon.

7. Eine Verbindung der Formel (I)

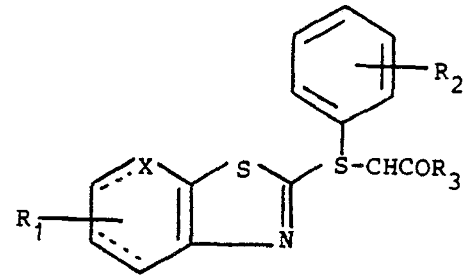

(I)

oder ein pharmakologisch annehmbares Salz hievon, worin $R_1$, $R_2$, $R_3$, X und die strichlierten Linien wie in Anspruch 1 definiert sind, mit der Maßgabe, daß, wenn $R_3$ Hydroxy ist, $R_1$ Nitro bedeutet.

8. Eine Verbindung, wie in Anspruch 1 beansprucht, die ausgewählt ist aus α-[(6-Nitro-2-benzothiazolyl)-thio]-benzolessigsäure und den pharmakologisch annehmbaren Salzen hievon; α-[(5-Chlor-2-benzothiazolyl)-thio]-benzoläthanthiosäure-S-[2-(2-naphthalenylamino)-2-oxoäthyl]-ester; α-[(5-Chlor-2-benzothiazolyl)-thio]-benzolessigsäure-[2-(acetylamino)-äthyl]ester; α-[(5-Chlor-2-benzothiazolyl)-thio]-N-(2-hydroxyäthyl)-benzolacetamid; und α-(5-Chlor-2-benzothiazolylthio)-benzolessigsäure-äthylester.

9. Eine Verbindung, wie in Anspruch 7 beansprucht, die α-(5-Chlorbenzothiazol-2-ylthio)-α-phenylacetamid ist.

10. Verfahren zur Herstellung einer Verbindung der Formel

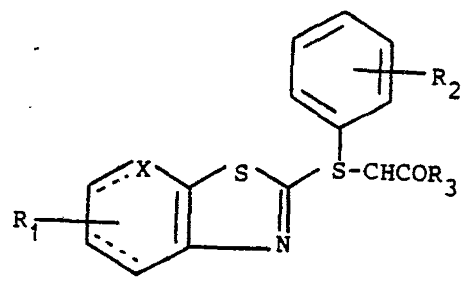

(I)

(worin $R_1$, $R_2$, $R_3$, X und die strichlierten Linien wie in Anspruch 1 definiert sind) oder eines pharmakologisch annehmbaren Salzes hievon, welches umfaßt:

(a) das Umsetzen einer Verbindung der Formel (II)

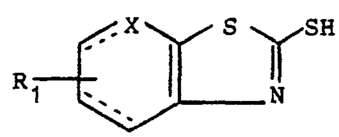

(II)

(worin $R_1$, X und die strichlierten Linien wie in Anspruch 1 definiert sind) oder eines Salzes hievon mit einer Verbindung der Formel

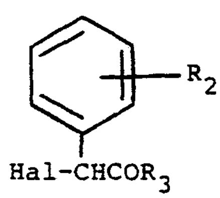

(III)

(worin Hal Chlor oder Brom ist und $R_2$ und $R_3$ wie in Anspruch 1 definiert sind, mit der maßgabe, daß, wenn $R_3$ Hydroxy ist, $R_1$ in Formel (II) Nitro bedeutet) oder einem Salz hievon; oder

(b) das Umsetzen einer Verbindung der Formel (IV)

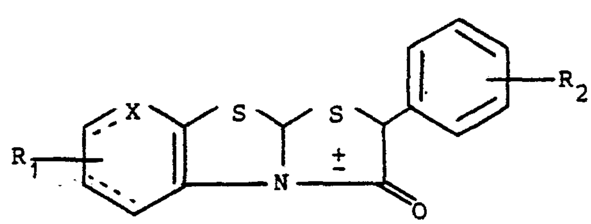

(IV)

mit einer Verbindung der Formel $R_3H$ (worin $R_1$, $R_2$, $R_3$, X und die strichlierten Linien wie in Anspruch 1 definiert sind).

**Revendications pour les Etats contractants: BE CH DE FR IT LI NL SE**

1. Utilisation d'un composé de formule

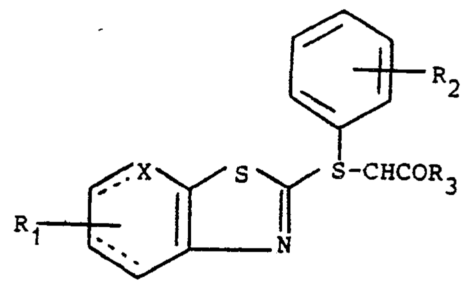

(I)

(dans laquelle $R_1$ est l'hydrogène, un radical halogéno, nitro, amino, alkyle en $C_1$ à $C_6$, alkoxy en $C_1$ à $C_6$, trifluorométhyle ou hydroxy; $R_2$ est l'hydrogène, le fluor, le chlore, le brome, un radical alkyle en $C_1$ à $C_6$, alkoxy en $C_1$ à $C_6$, amino, nitro ou trifluorométhyle; $R_3$ est un radical hydroxy, alkoxy en $C_1$ à $C_6$, amino, hydroxy(alkyle en $C_1$ à $C_6$)amino, N-(alcanoyle en $C_2$ à $C_7$)amino(alkoxy en $C_1$ à $C_6$) ou N-arylcarbamoyl-(alkyle en $C_1$ à $C_6$)thio; X représente CH ou N, et les segments en traits interrompus représentent des doubles liaisons facultatives dans les positions indiquées) ou d'un sel pharmacologiquement acceptable de ce composé pour la préparation d'un médicament destiné à être utilisé comme agent immunomodulateur.

2. Utilisation suivant la revendication 1, dans laquelle le noyau contenant X est un noyau iso-phénylène ou o-phénylène substitué par un halogène, un radical hydroxy ou alkoxy en $C_1$ à $C_6$, $R_2$ est l'hydrogène ou un radical alkyle en $C_1$ à $C_6$ et $R_3$ est un radical hydroxy, alkoxy en $C_1$ à $C_6$ ou $-NHCH_2CH_2OH$.

3. Utilisation suivant la revendication 1, dans laquelle le composé répondant à la formule I est choisi entre l'acide α-(2-benzothiazolylthio)benzène-acétique; l'acide α-(5-chlorobenzothiazole-2-ylthio)benzène-acètique; l'ester éthylique de l'acide α-(5-chloro-2-benzothiazolylthio)benzène-acétique; et l'α-[(5-chloro-2-benzothiazolyl)thio]-N-(2-hydroxyéthyl)benzene-acétamide.

4. Procédé de préparation d'un composé répondant à la formule

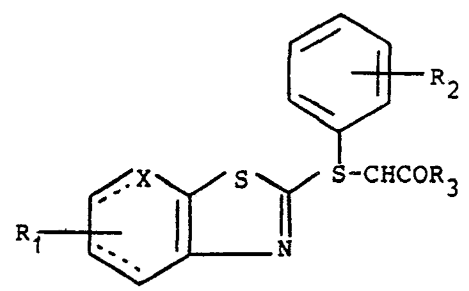

(I)

(dans laquelle $R_1$, $R_2$, $R_3$, X et les segments en traits interrompus sont tels que définis dans la revendication 1) ou d'un sel pharmacologiquement acceptable de ce composé, qui consiste à faire réagir un composé de formule IV

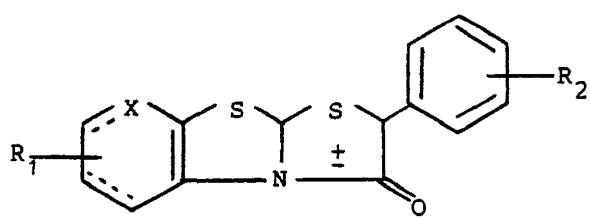

(IV)

avec un composé répondant à la formule $R_3H$ (formules dans lesquelles $R_1$, $R_2$, $R_3$, X et les segments en traits interrompus sont tels que définis dans la revendication 1).

5. Procédé suivant la revendication 4, dans lequel si $R_3$ est un radical hydroxy, $R_1$ est un radical nitro.

6. Composé de formule

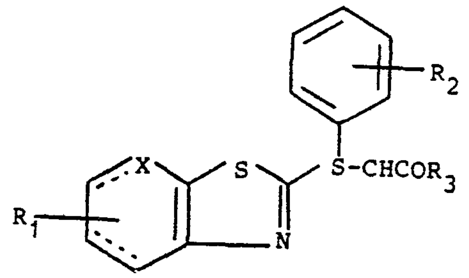

(I)

(dans laquelle $R_1$, $R_2$, $R_3$, X et les segments en traits interrompus sont tels que définis dans la revendication 1, sous réserve que (a) si $R_3$ est un radical hydroxy, $R_1$ soit un radical nitro et que (b) si $R_3$ est un radical alkoxy en $C_1$ à $C_6$ ou $—NHCH_2CH_2OH$ cependant que $R_2$ est l'hydrogène ou un radical alkyle en $C_1$ à $C_6$, le noyau contenant X ne soit pas un noyau o-phénylène ou o-phénylène substitué par un halogène ou un radical hydroxy ou alkoxy en $C_1$ à $C_6$), ou un sel pharmacologiquement acceptable de ce composé.

7. Composé suivant la revendication 6, le composé de formule I étant choisi entre l'α-(5-chlorobenzothiazole-2-ylthio)-α-phénylacétamide; l'ester 2-(acétylamino)éthylique de l'acide α-[(5-chloro-2-benzothiazolyl)thio]benzène-acétique; l'ester S-[2-(2-naphtalénylamino)-2-oxoéthylique de l'acide α-[(5-chloro-2-benzothiazolyl)thio]benzène-éthanethioïque et l'acide α-[(6-nitro-2-benzothiazolyl)thio]benzèneacétique.

8. Composition pharmaceutique, qui comprend un composé de formule I suivant la revendication 1 (dans laquelle $R_1$, $R_2$, $R_3$, X et les segments en traits interrompus sont tels que définis dans la revendication 1, sous réserve que lorsque le noyau contenant X est un noyau o-phénylène ou o-phénylène substitué par un halogéne, un radical hydroxy ou alkoxy en $C_1$ à $C_6$, cependant que $R_2$ est l'hydrogène ou un radical alkyle en $C_1$ à $C_6$, $R_3$ ne soit pas un radical hydroxy, alkoxy en $C_1$ à $C_6$ ou $—NHCH_2CH_2OH$) ou un sel pharmacologiquement acceptable de ce composé en association ou en mélange avec un support acceptable du point de vue pharmaceutique.

9. Composition suivant la revendication 8, dans laquelle le composé répondant à la formule I est choisi entre l'acide α-(5-chlorobenzothiazole-2-ylthio)-α-(*p*-chlorophényl)acétique; l'acide α-(*p*-chlorophényl)-α-(benzothiazole-2-ylthio)acétique; l'acide α-(5-chlorobenzothiazole-2-ylthio)-α-phénylacétamide; l'ester [2-(acétylamino)éthylique] d'acide α-[(5-chloro-2-benzothiazolyl)thio]benzène-acétique; l'ester S-[2-(2-naphtalénylamino)-2-oxoéthylique] de l'acide α-[(5-chloro-2-benzothiazolyl)thio]benzène-éthanethioïque; l'acide α-[(5-chlorothiazolo[5,4-*b*]pyridine-2-yl)thio]-α-phénylacétique; l'acide α-[5-chlorothiazolo[5,4-*b*]pyridine-2-yl)thio]-α-(*p*-chlorophényl)acétique; l'acide α-phényl-α-[(4,5,6,7-tétrahydrobenzothiazole-2-yl)thio]acétique; et l'acide α-[(6-nitro-2-benzothiazolyl)thio]benzène-acétique.

10. Composé destiné à être utilisé comme substance pharmaceutique, répondant à la formule I suivant la revendication 1 (dans laquelle $R_1$, $R_2$, $R_3$, X et les segments en traits interrompus sont tels que définis dans la revendication 1, sous réserve que (a) $R_3$ soit un radical hydroxy, (b) $R_1$ ne soit pas un radical nitro et (c) si le noyau contenant X est un noyau o-phénylène ou o-phénylène substitué par un halogène, un radical hydroxy ou alkoxy en $C_1$ à $C_6$, $R_2$ ne soit pas l'hydrogène ou un radical alkyle en $C_1$ à $C_6$) ou un sel pharmacologiquement acceptable de ce composé.

11. Composé suivant la revendication 10, le composé de formule I étant choisi entre l'acide α-(5-chlorobenzothiazole-2-ylthio)-α-(*p*-chlorophényl)acétique; l'acide α-(*p*-chlorophényl)-α-(benzothiazole-2-ylthio)-acétique; l'acide α-[(5-chlorothiazolo)[5,4-*b*]pyridine-2-yl)thio]-α-phénylacétique; l'acide α-[5-(chlorothiazolo)[5,4-*b*]pyridine-2-yl)thio]-α-(*p*-chlorophényl)acétique; et l'acide α-phényl-α-[(4,5,6,7-tétrahydrobenzothiazole-2-yl)thio]acétique.

**Revendications pour l'Etat contractant: AT**

1. Utilisation d'un composé de formule

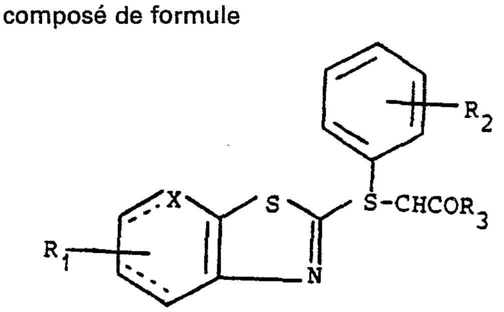

(I)

(dans laquelle $R_1$ est l'hydrogène, un radical halogéno, nitro, amino, alkyle en $C_1$ à $C_6$, alkoxy en $C_1$ à $C_6$, trifluorométhyle ou hydroxy; $R_2$ est l'hydrogène, le fluor, le chlore, le brome, un radical alkyle en $C_1$ à $C_6$, alkoxy en $C_1$ à $C_6$, amino, nitro ou trifluorométhyle; $R_3$ est un radical hydroxy, alkoxy en $C_1$ à $C_6$, amino,

hydroxy(alkyle en $C_1$ à $C_6$)amino, N-(alcanoyle en $C_2$ à $C_7$)amino(alkoxy en $C_1$ à $C_6$) ou N-arylcarbamoyl-(alkyle en $C_1$ à $C_6$)thio; X represente CH ou N, et les segments en traits interrompus représentent des doubles liaisons facultatives dans les positions indiquées) ou d'un sel pharmacologiquement acceptable de ce composé pour la préparation d'un médicament destiné à être utilisé comme agent immunomodulateur.

2. Utilisation suivant la revendication 1, dans laquelle le noyau contenant X est un noyau iso-phénylène ou o-phénylène substitué par un halogène, un radical hydroxy ou alkoxy en $C_1$ à $C_6$, $R_2$ est l'hydrogène ou un radical alkyle en $C_1$ à $C_6$ et $R_3$ est un radical hydroxy, alkoxy en $C_1$ à $C_6$ ou $—NHCH_2CH_2OH$.

3. Utilisation suivant la revendication 1 ou 2, dans laquelle le médicament est préparé sous la forme d'un comprimé ou d'une capsule ou sous la forme d'une solution stérile contenant d'autres solutés.

4. Utilisation suivant l'une quelconque des revendications 1 à 3, dans laquelle le médicament est préparé sous une forme dosée unitaire.

5. Utilisation suivant l'une quelconque des revendications 1 à 4, dans laquelle le composé répondant à la formule I est choisi entre l'acide α-(2-benzothiazolylthio)benzène-acétique; l'acide α-(5-chlorobenzo-thiazole-2-ylthio)benzène-acètique; l'ester éthylique de l'acide α-(5-chloro-2-benzothiazolylthio)benzène-acétique; et l'α-[(5-chloro-2-benzothiazolyl)thio]-N-(2-hydroxyéthyl)benzene-acétamide.

6. Procédé de préparation d'un composé de formule

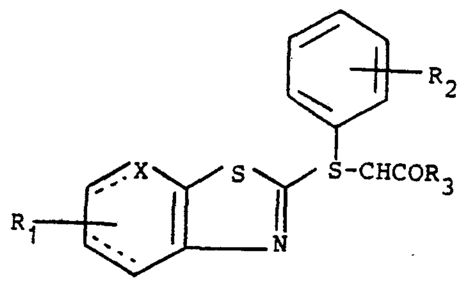

(I)

(dans laquelle $R_1$, $R_2$, $R_3$, X et les segments en traits interrompus sont tels que définis dans la revendication 1) ou d'un sel pharmacologiquement acceptable de ce composé, qui consiste à faire réagir un composé de formule IV

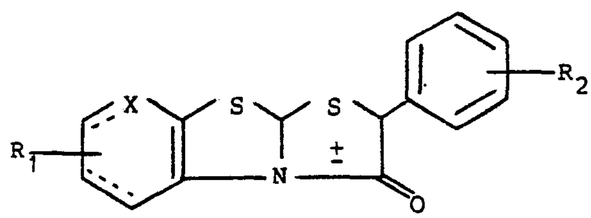

(IV)

avec un composé répondant à la formule $R_3H$ (formules dans lesquelles $R_1$, $R_2$, $R_3$, X et les segments en traits interrompus sont tels que définis dans la revendication 1).

7. Procédé suivant la revendication 6, dans lequel si $R_3$ est un groupe hydroxy, $R_1$ est un groupe nitro.

8. Procédé de préparation d'un composé de formule

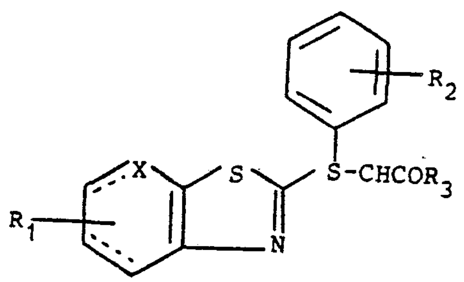

(I)

(dans laquelle $R_1$, $R_2$, $R_3$, X et les segments en traits interrompus sont tels que définis dans la revendication 1, sous réserve que (a) si $R_3$ est un groupe hydroxy, $R_1$ soit un groupe nitro et que (b) si $R_3$ est un groupe alkoxy en $C_1$ à $C_6$ ou $—NHCH_2CH_2OH$, tandis que $R_2$ est l'hydrogène ou un radical alkyle en $C_1$ à $C_6$, le noyau contenant X ne soit pas un noyau o-phénylène ou o-phénylène substitué par un halogène, un radical hydroxy ou alkoxy en $C_1$ à $C_6$), ou d'un sel pharmacologiquement acceptable de ce composé, procédé qui consiste à faire réagir un composé de formule II

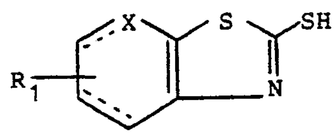

(II)

(dans laquelle $R_1$, X et les segments en traits interrompus sont tels que définis ci-dessus) ou un sel de ce composé avec un composé de formula

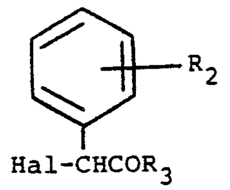

(III)

(dans laquelle Hal est le chlore ou le brome et $R_2$ et $R_3$ sont tels que définis ci-dessus) ou un sel de ce composé.

9. Procédé de préparation d'une composition pharmaceutique, qui consiste à mettre un composé de formule I suivant la revendication 1 (dans laquelle $R_1$, $R_2$, $R_3$, X et les segments en traits interrompus sont tels que définis dans la revendication 1, sous réserve que lorsque le noyau contenant X est un noyau o-phénylène ou o-phénylène substitué par un radical halogéno, hydroxy ou alkoxy en $C_1$ à $C_6$, tandis que $R_2$ est l'hydrogène ou un radical alkyle en $C_1$ à $C_6$, $R_3$ ne soit pas un radical hydroxy, alkoxy en $C_1$ à $C_6$ ou —$NHCH_2CH_2OH$) ou un sel pharmacologiquement acceptable de ce composé en association ou en mélange avec un support acceptable du point de vue pharmaceutique.

10. Procédé suivant la revendication 9, dans lequel le composé répondant à la formule I est choisi entre l'acide α-(5-chlorobenzothiazole-2-ylthio)-α-(*p*-chlorophényl)acétique; l'acide α-(*p*-chlorophényl)-α-(benzothiazole-2-ylthio)acétique; l'α-(5-chlorobenzothiazole-2-ylthio)-α-phénylacétamide; l'ester [2-(acétylamino)éthylique] de l'acide α-[(5-chloro-2-benzothiazolyl)thio]benzène-acétique; l'ester S-[2-(naphtalénylamino)-2-oxoéthylique] de l'acide α-[(5-chloro-2-benzothiazolyl)thio]benzène-éthanethioïque; l'acide α-[(5-chlorothiazolo[5,4-*b*]pyridine-2-yl)thio]-α-phenylacétique; l'acide α-[5-chlorothiazolo[5,4-*b*]pyridine-2-yl)thio-α-(*p*-chlorophényl)acétique; l'acide α-phényl-α-[(4,5,6,7-tétrahydrobenzothiazole-2-yl)-thio]acétique et l'acide α-[(6-nitro-2-benzothiazolyl)thio]benzène-acétique.

11. Procédé de préparation d'un composé de formule I suivant la revendication 8 ou d'un sel pharmacologiquement acceptable de ce composé destiné à être utilisé comme substance pharmaceutique, qui consiste à faire réagir un composé de formule II suivant la revendication 8 avec un composé de formule III suivant la revendication 8, formules dans lesquelles $R_1$, $R_2$, $R_3$, X et les segments en traits interrompus sont tels que définis dans la revendication 1, sous réserve que (a) $R_3$ soit un radical hydroxy, $R_1$ ne soit pas un radical nitro et (b) si $R_2$ est l'hydrogène ou un radical alkyle en $C_1$ à $C_6$, le noyau contenant X ne soit pas un noyau o-phénylène ou o-phénylène substitué par un halogène ou un radical hydroxy ou alkoxy en $C_1$ à $C_6$.

**Revendications pour l'Etat contractant: LU**

1. Composition pharmaceutique, comprenant un composé de formule I

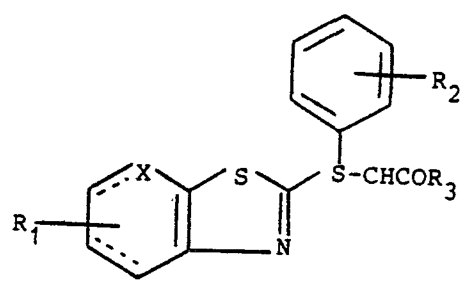

(I)

dans laquelle $R_1$ représente l'hydrogène, un radical halogéno, nitro, amino, alkyle en $C_1$ à $C_6$, alkoxy en $C_1$ à $C_6$, trifluorométhyle ou hydroxy; $R_2$ est l'hydrogène, le fluor, le chlore, le brome, un radical alkyle en $C_1$ à $C_6$, alkoxy en $C_1$ à $C_6$, amino, nitro ou trifluorométhyle; $R_3$ est un radical hydroxy, alkoxy en $C_1$ à $C_6$, amino, hydroxy-(alkyle en $C_1$ à $C_6$)amino, N-(alcanoyle en $C_2$ à $C_7$)amino(alkoxy en $C_1$ à $C_6$) ou N-arylcarbamoyl(alkyle en $C_1$ à $C_6$)thio; X représente CH ou N et les segments en traits interrompus représentent des doubles liaisons facultatives dans les positions 5, 6 et 7, 8 indiquées, ou un sel pharmacologiquement acceptable de ce composé en association ou en mélange avec un support pharmaceutiquement acceptable.

2. Composition suivant la revendication 1, sous une forme dosée unitaire.

3. Composition suivant la revendication 1 ou 2, sous la forme d'un ou plusieurs comprimés ou capsules ou sous la forme d'une solution stérile contenant d'autres solutés.

4. Composition suivant l'une quelconque des revendications 1 à 3, dans laquelle le composé de formule I est choisi entre l'acide α-(2-benzothiazolylthio)benzène-acétique; l'acide α-(5-chlorobenzothiazole-2-ylthio)benzène-acétique; l'acide α-(5-chlorobenzothiazole-2-ylthio)-α-(*p*-chlorophényl)acetique; l'acide α-(*p*-chlorophényl)-α-(benzothiazole-2-ylthio)acétique; l'ester éthylique de l'acide α-(5-chloro-2-benzothiazolylthio)benzène-acétique; l'α-(5-chlorobenzothiazole-2-ylthio)-α-phénylacétamide; l'α-[(5-chloro-2-benzothiazolyl)thio]-N-(2-hydroxyéthyl)benzène-acétamide; l'ester 2-(acétylamino)éthylique de l'acide α-[(5-chloro-2-benzothiazolyl)thio]benzène-acétique; l'ester S-[2-(2-naphtalénylamino)-2-oxo-éthylique] de l'acide α-[(5-chloro-2-benzothiazolyl)thio]benzène-éthanethioïque; l'acide α-[(5-chlorothiazolo[5,4-*b*]pyridine-2-yl)thio]-α-phénylacétique, l'acide α-[5-chlorothiazolo[5,4-*b*]pyridine-2-yl)thio]-α-(*p*-chlorophényl)acétique; l'acide α-phényl-α-[(4,5,6,7-tétrahydrobenzothiazole-2-yl)thio]-acétique; et l'acide α-[(6-nitro-2-benzothiazolyl)thio]benzène-acétique.

5. Composé destiné à être utilisé comme agent immunomodulateur, de formule I

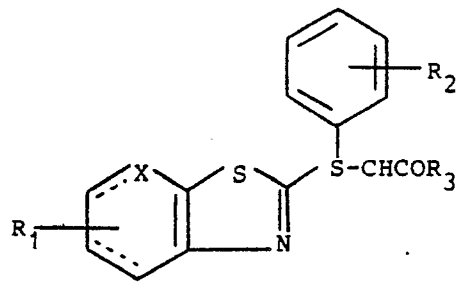

(I)

(dans laquelle $R_1$, $R_2$, $R_3$, X et les segments en traits interrompus sont tels que définis dans la revendication 1), ou un sel pharmacologiquement acceptable de ce composé.

6. Composé suivant la revendication 5, qui est choisi entre l'acide α-(2-benzothiazolylthio)benzène-acétique; l'acide α-(5-chlorobenzothiazole-2-ylthio)benzène-acétique; l'acide α-(5-chlorobenzothiazole-2-ylthio)-α-(*p*-(chlorophényl)acétique; l'acide α-(*p*-chlorophényl)-α-(benzothiazole-2-ylthio)acétique; l'acide 1-[(5-chlorothiazolo[5,4-*b*]pyridine-2-yl)thio]-α-phénylacétique; l'acide α-(5-chlorothiazolol[5,4-*b*]pyridine-2-yl)thio-α-(*p*-chlorophényl)acétique; l'acide α-phényl-α-[(4,5,6,7-tétrahydrobenzothiazole-2-yl)thio]-acétique et leurs sels pharmacologiquement acceptables.

7. Composé de formule I

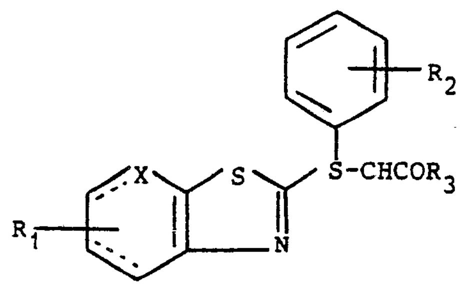

(I)

ou un sel pharmacologiquement acceptable de ce composé, formule dans laquelle $R_1$, $R_2$, $R_3$, X et les segments en traits interrompus sont tels que définis dans la revendication 1, sous réserve que lorsque $R_3$ est un groupe hydroxy, $R_1$ soit un groupe nitro.

8. Composé suivant la revendication 7, qui est choisi entre l'acide α-[(6-nitro-2-benzothiazolyl)thio]-benzène-acétique et ses sels pharmacologiquement acceptables; l'ester S-[2-(2-naphtalénylamino)-2-oxo-éthylique] de l'acide α-[(5-chloro-2-benzothiazolyl)thio]benzène-éthanethioïque; l'ester [2-(acétylmino)-éthylique] de l'acide α-[(5-chloro-2-benzothiazolyl)thio]benzène-acétique; l'α-[(5-chloro-2-benzo-thiazolyl)thio]-N-(2-hydroxyéthyl)benzène-acétamide et l'ester éthylique de l'acide α-(5-chloro-2-benzo-thiazolylthio)benzène-acétique.

9. Composé suivant la revendication 7, qui est l'α-(5-chlorobenzothiazole-2-ylthio)-α-phénylacétamide.

10. Procédé de préparation d'un composé de formule:

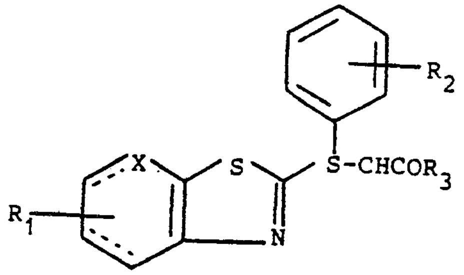

(I)

(dans laquelle $R_1$, $R_2$, $R_3$, X et les segments en traits interrompus sont tels que définis dans la revendication 1) ou d'un sel pharmacologiquement acceptable de ce composé, procédé qui consiste

(a) à faire réagir un composé de formule II

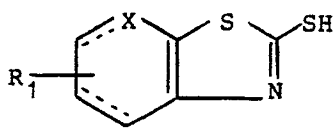

(II)

(dans laquelle $R_1$, X et les segments en traits interrompus sont tels que définis dans la revendication 1) ou un sel de ce composé avec un composé de formule

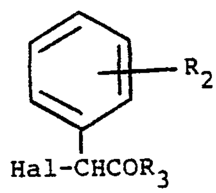

(III)

(dans laquelle Hal est le chlore ou le brome et $R_2$ et $R_3$ sont tels que définis dans la revendication 1, sous réserve que lorsque $R_3$ est un groupe hydroxy, $R_1$ dans la formule II soit un groupe nitro) ou un sel ce composé; ou bien

(b) à faire réagir un composé de formule IV

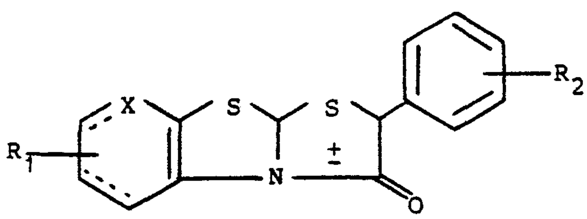

(IV)

avec un composé de formule $R_3H$ (formules dans lesquelles $R_1$, $R_2$, $R_3$, X et les segments en traits interrompus sont tels que définis dans la revendication 1).